(19) Europäisches Patentamt
European Patent Office
Office européen des brevets

(11) Veröffentlichungsnummer : **0 106 284**
**B1**

(12) # EUROPÄISCHE PATENTSCHRIFT

(45) Veröffentlichungstag der Patentschrift : **16.06.87**

(51) Int. Cl.⁴ : **C 07 D219/08**, A 61 K 31/435// C07D219/06

(21) Anmeldenummer : **83110035.9**

(22) Anmeldetag : **07.10.83**

(54) **3-Aryl-7-chlor-3,4-dihydro-acridin-1,9-(2H,10H)-dion-1-oxime und -1-hydrazonderivate, deren Salze, Verfahren zu ihrer Herstellung, sie enthaltende Mittel und ihre Verwendung.**

(30) Priorität : **11.10.82 DE 3237649**
**24.12.82 DE 3247908**

(43) Veröffentlichungstag der Anmeldung : **25.04.84 Patentblatt 84/17**

(45) Bekanntmachung des Hinweises auf die Patenterteilung : **16.06.87 Patentblatt 87/25**

(84) Benannte Vertragsstaaten :
**AT BE CH DE FR GB IT LI LU NL SE**

(56) Entgegenhaltungen :
**EP-A- 0 036 718**
**DE-A- 2 337 474**

(73) Patentinhaber : **HOECHST AKTIENGESELLSCHAFT**
**Postfach 80 03 20**
**D-6230 Frankfurt am Main 80 (DE)**

(72) Erfinder : **Winkelmann, Erhardt, Dr.**
**Brunhildenweg 5**
**D-6233 Kelkheim (Taunus) (DE)**
Erfinder : **Dürckheimer, Walter, Dr.**
**Im Lerchenfeld 45**
**D-6234 Hattersheim am Main (DE)**
Erfinder : **Raether, Wolfgang, Dr.**
**Falkensteinstrasse 6**
**D-6072 Dreieich (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 106 284 B1**

Jouve, 18, rue St-Denis, 75001 Paris, France

**Beschreibung**

In der DE-PS 2 337 474 werden 3-Aryl-7-chlor-10-hydroxy-3,4-dihydro-acridin-1,9-(2H, 10H)-dione beschrieben, die zur Bekämpfung von Protozoen, insbesondere Plasmodien (Malaria) und Coccidien geeignet sind. In der Europäischen Patentanmeldung mit der Veröffentlichungsnummer 36 718 werden auch gegen Protozoen wirksame 3-Aryl-7-chlor-10-hydroxy-3,4-dihydro-acridin-1,9-(2H, 10H)-dion-1-imine beschrieben.

Die Erfindung betrifft 3-Aryl-7-chlor-3,4-dihydroacridin-1,9-(2H, 10H)-dion-1-oxime und -1-hydrazonderivate der Formel I

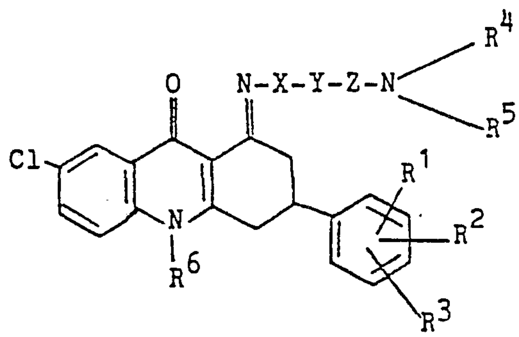

(I)

und ihre physiologisch verträglichen Säureadditions- und Ammoniumsalze.

In der Formel bedeuten

$R^1$, $R^2$ und $R^3$ Wasserstoff, Halogen, insbesondere Fluor, Chlor oder Brom, $C_1$-$C_4$-Alkyl, insbesondere Methyl, Trifluormethyl, $C_1$-$C_4$-Alkoxy, insbesondere Methoxy, Trifluormethoxy, Difluorchlormethoxy, 1,1,2,2-Tetrafluoräthoxy, Phenoxy, Halogenphenoxy, insbesondere Chlorphenoxy, $C_1$-$C_4$-Alkyl-, Phenyl- und Naphthyl-thio, insbesondere Methylthio und Phenylthio, $C_1$-$C_4$-Alkyl-, Phenyl- und Naphthyl-sulfinyl, insbesondere Methylsulfinyl und Phenylsulfinyl, $C_1$-$C_4$-Alkyl-, Phenyl- und Naphthyl-sulfonyl, insbesondere Methylsulfonyl und Phenylsulfonyl, Trifluormethylthio, Carbamyl, Sulfamyl, Cyano oder Nitro, wobei $R^1$, $R^2$ und $R^3$ gleich oder verschieden sind

a) X Sauerstoff

Y eine einfache Bindung

Z eine gerade oder verzweigte Alkylenkette mit 2 bis 5 Kohlenstoffatomen, die gegebenenfalls mit einer Hydroxygruppe substituiert ist,

b) X, Y und Z eine einfache Bindung,

c) X die —NR-Gruppe, worin R Wasserstoff oder $C_1$-$C_4$-Alkyl bedeutet,

Y eine einfache Bindung

Z eine gerade oder verzweigte Alkylenkette mit 2 bis 5 Kohlenstoffatomen,

d) X die —NR-Gruppe, worin R Wasserstoff oder $C_1$-$C_4$-Alkyl bedeutet,

Y die Carbonyl-, Thiocarbonyl- oder Iminocarbonyl-Gruppe

Z eine einfach Bindung,

e) X die —NR-Gruppe, worin R Wasserstoff oder $C_1$-$C_4$-Alkyl bedeutet,

Y die Carbonamid-, Thiocarbonamid- oder Carbonamidin-Gruppe

Z eine gerade oder verzweigte Alkylenkette mit 2 bis 5 Kohlenstoffatomen

f) X die —NR-Gruppe, worin R Wasserstoff oder $C_1$-$C_4$-Alkyl bedeutet,

Y die Carbonylgruppe

Z eine gerade oder verzweigte Alkylenkette mit 1 bis 3 Kohlenstoffatomen

g) X die —NR-Gruppe, worin R Wasserstoff oder $C_1$-$C_4$-Alkyl bedeutet

Y die Carboxygruppe

Z eine gerade oder verzweigte Alkylenkette mit 2 bis 5 Kohlenstoffatomen,

$R^4$ und $R^5$ Wasserstoff oder geradkettiges oder verzweigtes $C_1$-$C_4$-Alkyl wie Methyl, Äthyl, Propyl, Isopropyl, Butyl, Isobutyl, wobei $R^4$ und $R^5$ gleich oder verschieden, jedoch nicht gleich-, zeitig Wasserstoff sind, oder $R^4$ und $R^5$ zusammen eine Alkylenkette mit 4 bis 6 Kohlenstoffatomen, die mit dem Stickstoffatom einen 5- bis 7-gliedrigen heterocyclischen Ring bilden, worin eine —$CH_2$-Gruppe durch ein weiteres Heteroatom aus der Gruppe Stickstoff, Sauerstoff oder Schwefel ersetzt sein kann, und wobei der heterocyclische Ring seinerseits substituiert sein kann mit einer gegebenenfalls mit einer Hydroxygruppe substituierten Alkylgruppe mit ein bis drei Kohlenstoffatomen wie Methyl, Äthyl, 2-Hydroxyäthyl, Propyl, oder mit Phenyl- oder Naphthyl-$C_1$-$C_3$-Alkyl, wie Benzyl, oder mit Phenyl oder Naphthyl gegebenenfalls substituiert mit Methyl, Methoxy, Chlor oder Trifluormethyl, wie Phenyl, Tolyl, Methoxyphenyl, Chlorphenyl, Trifluormethylphenyl, insbesondere Pyrrolidin, 2-Methyl-pyrrolidin, 2,5-Dimethylpyrrolidin, Piperidin, 2-Methylpiperidin, 4-Methyl-piperidin, 2,6-Dimethylmorpholin, Thiomorpholin, Piperazin, N-Methyl-piperazin, N-Äthyl-piperazin, N-Benzyl-piperazin, N-Phenyl-piperazin, N-4-Tolylpiperazin, N-4-Methoxyphenyl-piperazin, N-4-Chlorphenyl-piperazin oder N-3-Trifluormethylphenyl-piperazin, und

$R^6$ Wasserstoff oder die Hydroxygruppe.

Je nach Definition des Gliedes X—Y—Z haben die Substituenten in 1-Stellung der erfindungsgemäßen 3-Aryl-7-chlor-10-hydroxy-3,4-dihydro-acridin-1,9-(2H,10H)-dion-1-oxime und -1-hydrazonderivate folgende Formeln :

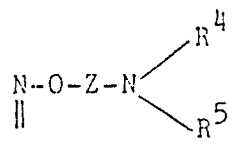 a)

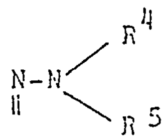 b)

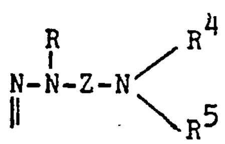 c)

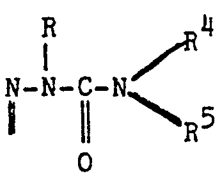 , 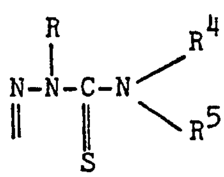 , 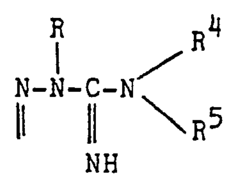 d)

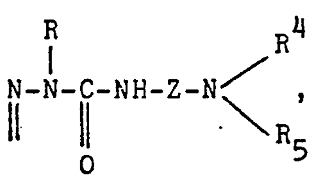 , 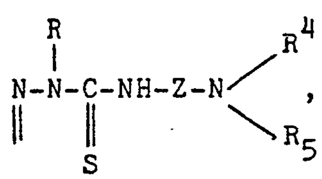 , 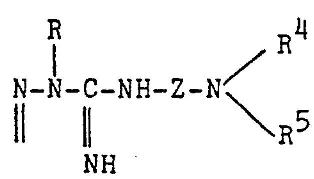 e)

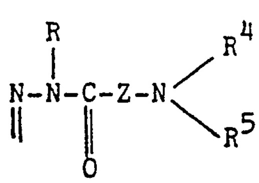 f)

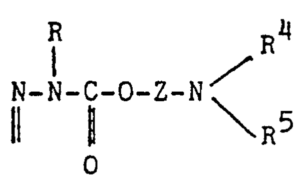 g)

Bevorzugt sind Verbindungen der Formel I, in welcher $R^1$ und $R^2$ Wasserstoff, $R^3$ Chlor oder Trifluormethyl, X, Y, Z eine einfache Bindung und $R^4$ und $R^5$ zusammen mit dem Stickstoffatom einen Piperazinring, der gegebenenfalls am Stickstoff substituiert ist, bedeuten. Ganz besonders bevorzugt

sind Verbindungen, die den Substituenten $R^3$ in 4-Stellung aufweisen.

Die Erfindung betrifft ebenso ein Verfahren zur Herstellung von 3-Aryl-7-chlor-3,4-dihydro-acridin-1,9-(2H, 10H)-dion-1-oximen und -1-hydrazonderivaten der Formel I und ihrer Salze, das dadurch gekennzeichnet ist, daß man ein 3-Aryl-7-chlor-3,4-dihydro-acridin-1,9-(2H, 10H)-dion der Formel II

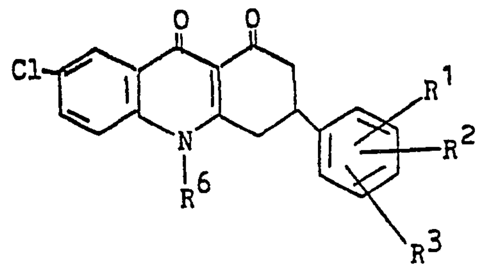

(II)

worin $R^1$, $R^2$, $R^3$ und $R^6$ die angegebene Bedeutung haben, mit einer Verbindung der Formel III

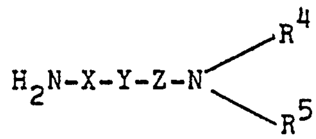

(III)

worin X, Y, Z sowie $R^4$ und $R^5$ die angegebene Bedeutung haben, oder deren Salzen umsetzt und das Reaktionsprodukt gegebenenfalls mit einer physiologisch verträglichen Säure in das betreffende Säureadditionssalz oder mit einem Alkylierungsmittel in das betreffende Ammoniumsalz überführt.

Die Ausgangsstoffe der Formel II sind entweder bekannt (vgl. z. B. DE-PS 2 337 474 und Arzneimittelforschung, Drug Research, 30 (II), Heft 7, S. 1041-46 (1980)) oder werden in analoger Weise wie in DE-PS 2 337 474 beschrieben durch Umsetzung (Reduktion) von 3-Aryl-7-chlor-10-hydroxy-3,4-dihydro-acridin-1,9-(2H, 10H)-dionen mit Phosphortrihalogeniden (Phosphortrichlorid) oder durch Umsetzung von 5-Chlor-antrhanilsäure bzw. deren Derivaten, wie z. B. Estern oder Anhydriden mit 5-Aryl-cyclohexan-1,3-dionen, hergestellt.

Die Ausgangsstoffe der Formel III sind ebenfalls entweder bekannt oder werden nach bekannten in der Fachliteratur beschriebenen Methoden hergestellt (vgl. z. B. Houben-Weyl, Methoden der Organischen Chemie, Georg Thieme Verlag, Stuttgart).

Als Ausgangsstoffe der Formel II kommen beispielsweise in Frage :

3-(2-,3-,4-Fluor) phenyl, 3-(2-,3-,4-Chlor)phenyl-,
3-(2-,3-,4-Brom)phenyl, 3-(2-,3-,4-Jod)phenyl-,
3-(2,4-,3,4-,2,5-,2,6-Dichlor)phenyl-,
3-(2-Fluor-4-chlor)phenyl-,
3-Phenyl-, 3-(2-,3-4-Methyl)phenyl-,
3-(2-Methyl-4-chlor)phenyl-, 3-(3-Methyl-4-chlor)phenyl-,
3-(2-,3-,4-Trifluormethyl)phenyl-, 3-(3,5-bis-Trifluormethyl) phenyl-,
3-(2-Chlor-4-trifluormethyl)phenyl-,
3-(2-,3-,4-Methoxy)phenyl-, 3-(3,4-,3,5-Dimethoxy)phenyl-,
3-(2-Chlor-4-methoxy)phenyl-,
3-(2-,3-,4-Trifluormethoxy)phenyl-,
3-(2-,3-,4-Difluorchlormethoxy)phenyl-,
3-(2-,3-,4-(1,1,2,2-Tetrafluoräthoxy))-phenyl-,
3-(2-,3-,4-Phenoxy)phenyl, 3-(2-,3-,4-(4-Chlorphenoxy))phenyl-,
3-(2-,3-,4-Methylthio)phenyl-, 3-(2-,3-,4-Phenylthio)phenyl-,
3-(2-,3-,4-Methylsulfinyl)phenyl-,
3-(2-,3-,4-Methylsulfonyl)phenyl-,
3-(2-,3-,4-Phenylsulfinyl)phenyl-,
3-(2-,3-,4-Phenylsulfonyl)phenyl-,
3-(2-,3-,4-Trifluormethylthio)phenyl-,
3-(2-,3-,4-Carbamyl)phenyl-, 3-(2-,3-,4-Sulfamyl)phenyl-
3-(2-,3-,4-Cyano)phenyl-, 3-(2-,3-,4-Nitro)phenyl-,
-7-chlor-10-hydroxy-3,4-dihydro-acridin-1,9-(2H,10H)-dion
bzw. -7-chlor-3,4-dihydro-acridin-, 1,9-(2H,10H)-dion.

Als Ausgangsstoffe der Formel III kommen beispielsweise in Frage :

a) O-(2-Aminoäthyl-, 2-Aminopropyl-, 3-Aminopropyl-, 4-Amino-butyl)-
O-(2-N-Methylaminoäthyl-, 2-N-Methylaminopropyl-, 3-N-Methylaminopropyl-, 4-N-Methylaminobu-

tyl)-

O-(2-N-Äthylaminoäthyl-, 2-N-Äthylaminopropyl-, 3-N-Äthylaminopropyl-, 4-N-Äthylaminobutyl)-

O-(2-N-n-Propylaminoäthyl-, 2-N-n-Propylaminopropyl-, 3-N-n-Propylaminopropyl-, 4-N-n-Propylaminobutyl)-

O-(2-N-n-Butylaminoäthyl-, 2-N-n-Butylaminopropyl-, 3-N-n-Butylaminopropyl-, 4-N-n-Butylaminobutyl)-

O-(2-N-Dimethylaminoäthyl-, 2-N-Dimethylaminopropyl-, 3-N-Dimethylaminopropyl-, 4-N-Dimethylaminobutyl)-

O-(2-N-Diäthylaminoäthyl-, 2-N-Diäthylaminopropyl-, 3-N-Diäthylaminopropyl-, 4-N-Diäthylaminobutyl)-

O-(2-N-Di-n-propylaminoäthyl-, 2-N-Di-n-propylaminopropyl-,
3-N-Di-n-propylaminopropyl-, 4-N-Di-n-propylaminobutyl)-

O-(2-N-Di-n-butylaminoäthyl-, 2-N-Di-n-butylaminopropyl-,
3-N-Di-n-butylaminopropyl-, 4-N-Di-n-butylaminobutyl)-

O-(2-Pyrrolidinoäthyl-, 2-Pyrrolidinopropyl-, 3-Pyrrolidinopropyl-, 4-Pyrrolidinobutyl)-

O-(2-Piperidinoäthyl-, 2-Piperidinopropyl-, 3-Piperidino-propyl-, 4-Piperidinobutyl)-

O-(2-Morpholinoäthyl-, 2-Morpholinopropyl-, 3-Morpholino-propyl-, 4-Morpholinobutyl)-

O-(2-Thiomorpholinoäthyl-, 2-Thiomorpholinopropyl-, 3-Thiomormolinopropyl-, 4-Thiomorpholinobutyl)-

O-(2-Piperazinoäthyl-, 2-Piperazinopropyl-, 3-Piperazino-propyl-, 4-Piperazinobutyl)-

O-(2-N-Methylpiperazinoäthyl-, 2-N-Methylpiperizinopropyl-,
3-N-Methylpiperazinopropyl-, 4-N-Methylpiperazinobutyl)-

O-(2-N-Äthylpiperazinoäthyl-, 2-N-Äthylpiperizinopropyl-,
3-N-Äthylpiperazinopropyl-, 4-N-Äthylpiperazinobutyl)-

O-(2-N-Benzylpiperazinoäthyl-, 2-N-Benzylpiperizinopropyl-,
3-N-Benzylpiperazinopropyl-, 4-N-Benzylpiperazinobutyl)-

O-(2-N-Phenylpiperazinoäthyl-, 2-N-Phenylpiperizinopropyl-,
3-N-Phenylpiperazinopropyl-, 4-N-Phenylpiperazinobutyl)-hydroxylamin

O-(3-Amino-, 3-Methylamino-3-N-Äthylamino, 3-N-n-Propylamino-, 3-N-n-Butylamino-, 3-N-Dimethylamino-, 3-N-Diäthylamino-, 3-N-Di-N-Propylamino-, 3-N-Di-n-Butylamino-, 3-Pyrrolidino-, 3-Piperidino-, 3-Morpholino-, 3-Thiomorpholino-, 3-Piperazino-, 3-N'-Methylpiperazino-, 3-N'-Äthylpiperazino-, 3-N'-Phenylpiperazino-2-hydroxy-propyl)-hydroxylamin

b) N-Methyl-, N-Äthyl-, N-n-Propyl-, N-n-Butyl, N,N-Dimethyl-N,N-Diäthyl-, N,N-Di-n-propyl-, N,N-Di-n-butyl-hydrazin, N-Amino-pyrrolidin, -piperidin, -morpholin, -thiomorpholin, -piperazin, -N'-Methylpiperazin, -N'-Äthylpiperazin, N'-2-Hydroxyäthylpiperazin, -N'-Benzylpiperazin, -N'-Phenylpiperazin, -hexamethylenimin, -N'-Methylhomopiperazin

N-Methylamino-, N-Äthylamino-, N-n-Propylamino, N-n-Butylamino-, N-Dimethylamino-, N-Diäthylamino-, N-Di-n-propylamino-, N-Di-n-butylamino-, Pyrrolidino-, Piperidino-, Morpholino-, Thiomorpholino-, Piperazino-, N'-Methylpiperazino-, N'-äthylpiperazino-, N'-Benzylpiperazino-, N'-Phenylpiperazino-äthyl-, -propyl-, -isopropyl-, -butyl-hydrazin

d) Hydrazinocarbonyl-, Hydrazinothiocarbonyl-, Hydrazino-carbimidopyrrolidin, -piperidin, -morpholin, -thiomorpholin, -piperazin, -N'-methylpiperazin, -N'-äthyl-piperazin, -N'-benzylpiperazin -N'-phenylpiperazin, -N'-2-hydroxyäthylpiperazin

e) 1-Methyl-, 1-Äthyl-, 1-Propyl-, 1-Butyl-, 1,1-Dimethyl-, 1,1-Diäthyl-, 1,1-Di-n-propyl-, 1,1-Di-n-butyl-, 1-N-Dimethylamino-, -Diäthylmino-, -Di-n-propylamino-, -Di-n-butylamino-, -Pyrrolidino-, -Piperidino-, -Morpholino-, -Thiomorpholino-, -Piperazino-, -N-Methylpiperazino-, -N-Äthylpiperazino-, -N-Benzylpiperazino-, -N-Phenyl-piperazino-2-äthyl-, -2-propyl-, -3-propyl-, -4-butyl-semicarbazid, -thiosemicarbazid, -aminoguanidin

f) Amino-, N-Methylamino-, N-Äthylamino-, N-n-Propylamino-, N-n-Butylamino-, N-Dimethylamino-, N-Diäthylamino-, N-Di-n-propylamino-, N-Di-n-butylamino-, Pyrrolidino-, Piperidino-, Morpholino-, Thiomorpholino-, Piperazino-, N'-Methylpiperazino-, N'-Äthylpiperazino-, N'-2-Hydroxy-äthylpiperazino-, N'-Benzylpiperazino-, N'-Phenylpiperazino-essigsäure-, -propionsäure-, -buttersäure, -isobuttersäure-hydrazid,

Trimethyl-, Triäthyl-, Tri-n-propyl-, Tri-n-butyl-ammonium-essigsäure-, propionsäure-, -buttersäure, -isobuttersäurehydrazid-chlorid, -bromid, -jodid

g) Hydrazinoameisensäure-amino-, -N-Methylamino-, -N-Äthylamino-, -N-n-Propylamino-, -N-n-Butylamino-, -N-Dimethylamino-, -N-Diäthylamino-, -N-Di-n-propylamino-, -N-Di-n-butylamino-, -pyrrolidino-, -piperidino-, -morpholino-, -thiomorpholino-, -piperazino-, -N'-Methylpiperazino-, -N'-Athylpiperazino -N'-Benzylpiperazino-, -N'-Phenylpiperazino-äthyl-, -propyl-, -isopropyl-, -butyl-ester

Die Umsetzung der Verbindungen der Formel II mit Verbindungen der Formel III erfolgt zweckmäßig in molaren Mengen. Die Umsetzungen werden vorteilhaft in einem Lösungs- oder Verteilungsmittel durchgeführt.

Als Lösungs- oder Verteilungsmittel kommen beispielsweise in Frage : Alkohole, wie Methanol, Äthanol, Propanol, Isopropanol, Butanol, Methoxyäthanol, Äthoxyäthanol, Amide, wie Dimethylformamid, Dimethylacetamid, ferner Dimethylsulfoxid, Halogenkohlenwasserstoffe, wie Methylenchlorid, Chloroform, 1,2-Dichloräthan, Äther, wie 1,2-Dimethoxyäthan, 1,2-Diäthoxyäthan, Tetrahydrofuran, Dioxan.

Die Reaktionstemperaturen liegen z. B. zwischen 40 und 120 °C, vorzugsweise zwischen 60 und 80 °C.

Die Reaktionszeiten betragen je nach Reaktionskomponente und je nach Temperaturbereich wenige Minuten bis einige Stunden.

Die bei der Umsetzung erhaltenen Reaktionsprodukte fallen meist als freie Basen an und können gegebenenfalls mit physiologisch verträglichen Säuren in die entsprechenden Salze übergeführt werden.

Als Säuren für die Salzbildung kommen beispielsweise in Frage : Halogenwasserstoffsäuren, insbesondere Salzsäure, ferner Schwefelsäure, Salpetersäure, Phosphorsäure, Essigsäure, Propionsäure, Milchsäure, Weinsäure, Zitronensäure, Oxalsäure, Bernsteinsäure, Maleinsäure, Fumarsäure, Sorbinsäure, Salicylsäure, Methyl-, Phenyl-, 4-Tolysulfonsäure oder 1,5-Naphthalindisulfonsäure.

Die freien Basen werden gegebenenfalls in die Ammoniumsalze überführt. Hierzu eignen sich insbesondere die $C_1$-$C_4$-Alkylhalogenide wie Methyl-, Äthyl-, Propyl-, Butyl-, i-Propyl-chlorid-, bromid und -jodid, sowie die Benzylhalogenide.

Setzt man als Ausgangsverbindungen der Formel III die Ammoniumsalze ein, so fallen die Verbindungen I als Ammoniumsalze an.

Die erfindungsgemäßen Verbindungen der Formel I haben wertvolle chemotherapeutische Eigenschaften und eignen sich besonders zur Bekämpfung von Infektionen durch Protozoen bei Mensch und Tier. Sie zeichnen sich beispielsweise durch eine hohe Wirkung gegenüber Plasmodien, den Erregern von Malaria, aus. Sie besitzen im Vergleich zu Verbindungen gemäß DE-PS 2 337 474 eine wesentlich stärkere Wirksamkeit. Das bedeutet, daß nach Verabreichung von wesentlich kleineren Dosierungen der gleich Effekt erreicht wird. Die Wirkung richtet sich vor allem gegen solche Plasmodien-Stämme, die auf herkömmliche Arzneimittel, wie z. B. Chloroquine und andere Aminochinoline nicht mehr im ausreichenden Maße ansprechen, d. h. resistent sind. Die neuen Verbindungen besitzen keine Kreuzresistenz zu bekannten Mitteln. Die beanspruchten Verbindungen sind auch gegen die Erreger der Coccidiose der Hühner, Puten, Kaninchen, Rinder und Schweine wirksam.

Die Erfindung betrifft daher auch pharmazeutische Präparate auf Basis der erfindungsgemäßen Verbindungen sowie ihre Verwendung als Arzneimittel, insbesondere als Chemotherapeutikum. Durch günstige physikalische Eigenschaften können die Verbindungen auch als Salze angewendet werden und ermöglichen neben der enteralen (oralen) Applikation auch eine parenterale Anwendung.

Die Verbindungen der Formel I oder ihre Salze können enteral oder parenteral zweckmäßig in Dosen von 0,5 bis 100 mg/kg Körpergewicht verabreicht werden. Sie sind auch in Kombination mit anderen Wirkstoffen verwendbar.

Sie können in Form von Flüssigkeiten, Pulvern, Tabletten, Kapseln appliziert werden. Die Verbindungen werden dazu mit Hilfsstoffen gemischt, beispielsweise mit pharmazeutisch üblichen, flüssigen oder festen Füllstoffen, Lösungsmitteln, Emulgatoren, Gleitstoffen, Geschmackskorrigentien, Farbstoffen und/oder Puffersubstanzen. Außerdem kann man die Verbindungen I auch in Mischung mit geeigneten Futtermitteln verabreichen.

A. Herstellungsbeispiele : ($R^6$ = OH)

Beispiel 1

3-(4-Trifluormethylphenyl)-7-chlor-10-hydroxy-3,4-dihydroacridin-1,9-(2H,10H)-dion-1-(N-methyl-N'-piperazin)-imin

40,75 g (0,1 Mol) 3-(4-Trifluormethylphenyl)-7-chlor-10-hydroxy-3,4-dihydro-acridin-1,9-(2H, 10H)-dion werden in 500 ml Äthanol suspendiert, 11,5 (0,1 Mol) N-Methyl-N'aminopiperazin werden zugefügt und die Reaktionsmischung wird 30 Minuten zum Rückfluß erhitzt. Dabei bildet sich eine klare orangerote Lösung. Nach dem Abkühlen wird am Rotationsverdampfer das Äthanol abdestilliert und der feste Rückstand aus Toluol-Cyclohexan (1 : 1) umkristallisiert. Man erhält so 43 g = 85 % der Theorie der Titelverbindung in Form von orangegelben Kristallen mit dem Fp. 213 °C.

Die Herstellung des Ausgangsstoffes 3-(4-Trifluormethylphenyl)-7-chlor-10-hydroxy-3,4-dihydro-acridin-1,9-(2H, 10H)-dion ist in DE-PS 2 337 474 beschrieben.

In analoger Weise wie in Beispiel 1 beschrieben werden folgende Verbindungen hergestellt :

a) 1. aus 3-(4-Trifluormethylphenyl)-7-chlor-10-hydroxy-3,4-dihydroacridin-1,9-(2H,10H)-dion = A (Herstellung vgl. DE-PS 2 337 474) und O-(2-N-Dimethylaminoäthyl)-hydroxylamin erhält man A-1-[O-(2-N-Dimethylaminoäthyl)-oxim] Fp. 235°

aus A und O-(3-N-Dimethylaminopropyl)-hydroxylamin
A-1-[O-(3-N-Dimethylaminopropyl)-oxim]

aus A und O-(2-N-Diäthylaminoäthyl)-hydroxylamin
A-1-[O-(2-N-Diäthylaminoäthyl)-oxim] Fp. 197°

aus A und O-(3-N-Diäthylaminopropyl)-hydroxylamin
A-1-[O-(3-N-Diäthylaminopropyl)-oxim]

aus A und O-(2-Pyrrolidinoäthyl)-hydroxylamin
A-1-[O-(2-Pyrrolidinoäthyl)-oxim] Fp. 203°

aus A und O-(2-Piperidinoäthyl)-hydroxylamin
A-1-[O-(2-Piperidinoäthyl)-oxim] Fp. 223°

aus A und O-(2-Morpholinoäthyl)-hydroxylamin
A-1-[O-(2-Morpholinoäthyl)-oxim] Fp. 248°

aus A und O-(2-Thiomorpholinoäthyl)-hydroxylamin
A-1-[O-(2-Thiomorpholinoäthyl)-oxim]

aus A und O-(2-N'-Methylpiperazinoäthyl)-hydroxylamin
A-1-[O-(2-N'-Methylpiperazinoäthyl)-oxim]

aus A und O-(2-N'-Benzylpiperazinoäthyl)-hydroxylamin
A-1-[O-(2-N'-Benzylpiperazinoäthyl)-oxim]

aus A und O-(2-N'-Phenylpiperazinoäthyl)-hydroxylamin
A-1-[O-(2-N'-Phenylpiperazinoäthyl)-oxim]

aus A und O-(3-N-Dimethylamino-2-hydroxypropyl)-hydroxylamin
A-1-[O-(3-N-Dimethylamino-2-hydroxypropyl)-oxim]

aus A und O-(3-N-Diäthylamino-2-hydroxypropyl)-hydroxylamin
A-1-[O-(3-N-Diäthylamino-2-hydroxypropyl)-oxim]

aus A und O-(3-Morpholino-2-hydroxypropyl)-hydroxylamin
A-1-[O-(3-Morpholino-2-hydroxypropyl)-oxim]

aus A und O-(3-N'-Methylpiperazino-2-hydroxypropyl)-hydroxylamin
A-1-[O-(3-N'-Methylpiperazino-2-hydroxypropyl)-oxim]

a) 2. aus 3-(4-Chlorphenyl)-7-chlor-10-hydroxy-3,4-dihydro-acridin-1,9-(2H, 10H)-dion = B (Herstellung nach DE-PS 2 337 474) und O-(2-N-Dimethylaminoäthyl)-hydroxylamin erhält man B-1-[O-(2-N-Dimethylaminoäthyl)-oxim] Fp. 250°

aus B und O-(3-N-Dimethylaminopropyl)-hydroxylamin
B-1-[O-(3-N-Dimethylaminopropyl)-oxim]

aus B und O-(2-N-Diäthylaminoäthyl)-hydroxylamin
B-1-[O-(2-N-Diäthylaminoäthyl)-oxim]

aus B und O-(3-N-Diäthylaminopropyl)-hydroxylamin
B-1-[O-(3-N-Diäthylaminopropyl)-oxim]

aus B und O-(2-Pyrrolidinoäthyl)-hydroxylamin
B-1-[O-(2-Pyrrolidinoäthyl)-oxim]

aus B und O-(2-Piperidinoäthyl)-hydroxylamin
B-1-[O-(2-Piperidinoäthyl)-oxim]

aus B und O-(2-Morpholinoäthyl)-hydroxylamin
B-1-[O-(2-Morpholinoäthyl)-oxim]

aus B und O-(2-Thiomorpholinoäthyl)-hydroxylamin

B-1-[O-(2-Thiomorpholinoäthyl)-oxim]

aus B und O-(2-N'-Methylpiperazinoäthyl)-hydroxylamin
B-1-[O-(2-N'-Methylpiperazinoäthyl)-oxim]

aus B und O-(2-N'-Benzylpiperazinoäthyl)-hydroxylamin
B-1-[O-(2-N'-Benzylpiperazinoäthyl)-oxim]

aus B und O-(2-N'-Phenylpiperazinoäthyl)-hydroxylamin
B-1-[O-(2-N'-Phenylpiperazinoäthyl)-oxim]

aus B und O-(3-N-Dimethylamino-2-hydroxypropyl)-hydroxylamin
B-1-[O-(3-N-Dimethylamino-2-hydroxypropyl)-oxim]

aus B und O-(3-N-Diäthylamino-2-hydroxypropyl)-hydroxylamin
B-1-[O-(3-N-Diäthylamino-2-hydroxypropyl)-oxim]

aus B und O-(3-Morpholino-2-hydroxypropyl)-hydroxylamin
B-1-[O-(3-Morpholino-2-hydroxypropyl)-oxim]

aus B und O-(3-N'-Methylpiperazino-2-hydroxypropyl)-hydroxylamin
B-1-[O-(3-N'-Methylpiperazino-2-hydroxypropyl)-oxim]

b) 1. aus 3-(4-Trifluormethylphenyl)-7-chlor-10-hydroxy-3,4-dihydro-acridin-1,9-(2H, 10H)-dion = A und N,N-Dimethylhydrazin
A-1-N,N-Dimethylhydrazon Fp. 202°

aus A und N,N-Di-butylhydrazin
A-1-N,N-Di-n-butylhydrazon ;

aus A und N-Aminopyrrolidin
A-1-N-Pyrrolidinimin ;

aus A und N-Aminomorpholin
A-1-N-Morpholinimin Fp. 243° ;

aus A und N-2-Hydroxyäthyl-N'-aminopiperazin
A-1-N-2-Hydroxyäthyl-N'-piperazinimin Fp. 210° ;

aus A und N-Benzyl-N'-aminopiperazin
A-1-N-Benzyl-N'-piperazinimin Fp. 240° ;

aus A und N-Phenyl-N'-aminopiperazin
A-1-N-Phenyl-N'-piperazinimin Fp. 268° ;

b) 2. aus 3-(4-Chlorphenyl)-7-chlor-10-hydroxy-3,4-dihydroacridin-1,9-(2H, 10H)-dion = B und N,N-dimethylhydrazin
B-1-N,N-Dimethylhydrazon ;

aus B und N,N-Di-n-butylhydrazin
B-1-N,N-butylhydrazon ;

aus B und N-Aminopyrrolidin
B-1-N-Pyrrolidinimin ;

aus B und N-Aminomorpholin
B-1-N-Morpholinimin ;

aus B und N-Methyl-N'-aminopiperazin
B-1-N-Methyl-N'-piperazinimin Fp. 228° ;

aus B und N-2-Hydroxyäthyl-N'-aminopiperazin
B-1-N-2-Hydroxyäthyl-N'-piperazinimin Fp. 225° ;

aus B und N-Benzyl-N'-aminopiperazin

B-1-N-Benzyl-N'-piperazinimin ;

aus B und N-Phenyl-N'-aminopiperazin
B-1-N-Phenyl-N'-piperazinimin ;

b) 3. aus 3-Phenyl-7-chlor-10-hydroxy-3,4-dihydro-acridin-1,9-(2H, 10H)-dion = D und N-Methyl-N'-aminopiperazin
D-1-N-Methyl-N'-piperazinimin Fp 118° ;

4. aus 3-(4-Fluorphenyl)-7-chlor-10-hydroxy-3,4-dihydro-acridin-1,9-(2H, 10H)-dion = E und N-Methyl-N'-amino-piperazin
E-1-N-Methyl-N'-piperazinimin Fp. 210° ;

5. aus 3-(2-Chlorphenyl)-7-chlor-10-hydroxy-3,4-dihydro-acridin-1,9-(2H, 10H)-dion = G und N-Methyl-N'-aminopiperazin
G-1-N-Methyl-N'-piperazinimin Fp. 147° ;

6. aus 3-(3-Chlorphenyl)-7-chlor-10-hydroxy-3,4-dihydro-acridin-1,9-(2H, 10H)-dion = L und N-Methyl-N'-aminopiperazin
L-1-N-Methyl-N'-piperazinimin Fp. 203° ;

7. aus 3-(2,4-Dichlorphenyl)-7-chlor-10-hydroxy-3,4-dihydro-acridin-1,9-(2H, 10H)-dion = M und N-Methyl-N'-aminopiperazin
M-1-N-Methyl-N'-piperazinimin Fp. 157° ;

8. aus 3-(3,4-Dichlorphenyl)-7-chlor-10-hydroxy-3,4-dihydro-acridin-1,9-(2H, 10H)-dion = Q und N-Methyl-N'-aminopiperazin
Q-1-N-Methyl-N'-piperazinimin Fp. 241° ;

9. aus 3-(2-Trifluormethylphenyl)-7-chlor-10-hydroxy-3,4-dihydro-acridin-1,9-(2H, 10H)-dion = T und N-Methyl-N'-aminopiperazin
T-1-N-Methyl-N'-piperazinimin Fp. 237° ;

10. aus 3-(2-Chlor-4-trifluormethylphenyl)-7-chlor-10-hydroxy-3,4-dihydro-acridin-1,9-(2H, 10H)-dion = U und N-Methyl-N'-aminopiperazin
U-1-N-Methyl-N'-piperazinimin Fp. 205° ;

11. aus 3-(4-Methoxyphenyl)-7-chlor-10-hydroxy-3,4-dihydro-acridin-1,9-(2H, 10H)-dion = V und N-Methyl-N'-aminopiperazin
V-1-N-Methyl-N'-piperazinimin Fp. 152° ;

12. aus 3-(4-Nitrophenyl)-7-chlor-10-hydroxy-3,4-dihydro-acridin-1,9-(2H, 10H)-dion = W und N-Methyl-N'-aminopiperazin
W-1-N-Methyl-N'-piperazinimin Fp. 217° ;

c) 1. aus A und 2-N-Dimethylaminoäthylhydrazin
A-1-2-N-Dimethylaminoäthylhydrazon ;

aus A und 2-N-Diäthylaminoäthylhydrazin
A-1-2-N-Diäthylaminoäthylhydrazon Fp. 180° ;

aus A und 2-Piperidinoäthylhydrazin
A-1-(2-Piperidinoäthyl)hydrazon ;

aus A und 2-N-Methylpiperazinoäthylhydrazin
A-1-(2-N-Methylpiperazinoäthyl)hydrazon ;

c) 2. aus B und 2- N-Dimethylaminoäthylhydrazin
B-1-2-N-Dimethylaminoäthylhydrazon ;

aus B und 2-N-Diäthylaminoäthylhydrazin
B-1-2-N-Diäthylaminoäthylhydrazon ;

aus B und 2-Piperidinoäthylhydrazin
B-1-(2-Piperidinoäthyl)hydrazon

aus B und 2-N-Methylpiperazinoäthylhydrazin
B-1-(2-N-methylpiperazinoäthyl)hydrazon ;

d) 1. aus 3-(4-Trifluormethylphenyl)-7-chlor-10-hydroxy-3,4-dihydro-acridin-1,9-(2H, 10H)-dion = A und 1,1-Dimethyl-semicarbazid
A-1-(1,1-Dimethyl)-semicarbazon ;

aus A und 1,1-Dimethyl-thiosemicarbazid
A-1-(1,1-Dimethyl)-thiosemicarbazon ;

aus A und 1,1-Dimethyl-aminoguanidin
A-1-(1,1-Dimethyl)-guanylhydrazon ;

aus A und N-Hydrazinocarbonyl-N'-methylpiperazin
A-1-(N'-Methylpiperazino-N-carbonyl)-hydrazon ;

aus A und N-Hydrazinothiocarbonyl-N'-methylpiperazin
A-1-(N'-Methylpiperazino-N-thiocarbonyl)-hydrazon Fp. 238° ;

aus A und N-hydrazinocarbimido-N'-methylpiperazin
A-1-(N'-Methylpiperazino-N-carbimido)-hydrazon ;

d) 2. aus 3-(4-Chlorphenyl)-7-chlor-10-hydroxy-3,4-dihydroacridin-1,9-(2H, 10H)-dion = B
und 1,1-Dimethyl-semicarbazid
B-1-(1,1-Dimethyl)-semicarbazon ;

aus B und 1,1-Dimethyl-thiosemicarbazid
B-1-(1,1-Dimethyl)-thiosemicarbazon ;

aus B und 1,1-Dimethyl-aminoguanidin
B-1-(1,1-Dimethyl)-guanylhydrazon ;

aus B und N-Hydrazinocarbonyl-N'-methylpiperazin
B-1-(N'-Methylpiperazino-N-carbonyl)-hydrazon ;

aus B und N-Hydrazinothiocarbonyl-N'-methylpiperazin
B-1-(N'-Methylpiperazino-N-thiocarbonyl)-hydrazon ;

aus B und N-Hydrazinothiocarbimido-N'-methylpiperazin
B-1-(N'-Methylpiperazino-N-carbimido)-hydrazon ;

e) 1. aus A und 1-(2-N-Dimethylaminoäthyl)-semicarbazid
A-1-[1-(2-N-Dimethylaminoäthyl)]-semicarbazon ;

aus A und 1-(2-N-Diäthylaminoäthyl)-semicarbazid
A-1-[1-(2-N-Diäthylaminoäthyl)]-semicarbazon ;

aus A und 1-(2-N-Methylpiperazinoäthyl)-semicarbazid
A-1-[1-(2-N-Methylpiperazinoäthyl)]-semicarbazon ;

aus A und 1-(2-N-Dimethylaminoäthyl)-thiosemicarbazid
A-1-[1-(2-N-Dimethylaminoäthyl)]-thiosemicarbazon ;

aus A und 1-(2-N-diäthylaminoäthyl)-thiosemicarbazid
A-1-[1-(2-N-Diäthylaminoäthyl)]-thiosemicarbazon Fp. 225° ;

aus A und 1-(2-N-Methylpiperazinoäthyl)-thiosemicarbazid
A-1-[1-(2-N-Methylpiperazinoäthyl)]-thiosemicarbazon ;

aus A und 1-(2-N-Dimethylaminoäthyl)-aminoguanidin
A-1-[1-(2-N-Dimethylaminoäthyl)]-guanylhydrazon ;

aus A und 1-(2-N-Diäthylaminoäthyl)-aminoguanidin
A-1-[1-(2-N-Diäthylaminoäthyl)]-guanylhydrazon ;

aus A und 1-(2-N-Methylpiperazinoäthyl)-aminoguanidin
A-1-[1-(2-N-Methylpiperazinoäthyl)]-guanylhydrazon ;

e) 2. aus B und 1-(2-N-Dimethylaminoäthyl)-semicarbazid
B-1-[1-(2-N-Dimethylaminoäthyl)]-semicarbazon ;

aus B und 1-(2-N-Diäthylaminoäthyl)-semicarbazid
B-1-[1-(2-N-Diäthylaminoäthyl)]-semicarbazon ;

aus B und 1-(2-N-Methylpiperazinoäthyl)-semicarbazid
B-1-[1-(2-N-Methylpiperazinoäthyl)]-semicarbazon ;

aus B und 1-(2-N-Dimethylaminoäthyl)-thiosemicarbazid
B-1-[1-(2-N-Dimethylaminoäthyl)]-thiosemicarbazon ;

aus B und 1-(2-N-Diäthylaminoäthyl)-thiosemicarbazid
B-1-[1-(2-N-diäthylaminoäthyl)]-thiosemicarbazon ;

aus B und 1-(2-N-Methylpiperazinoäthyl)-thiosemicarbazid
B-1-[1-(2-N-Methylpiperazinoäthyl)]-thiosemicarbazon ;

aus B und 1-(2-N-Dimethylaminoäthyl)-aminoguanidin
B-1-[1-(2-N-Dimethylaminoäthyl)]-guanylhydrazon ;

aus B und 1-(2-N-Diäthylaminoäthyl)-aminoguanidin
B-1-[1-(2-N-Diäthylaminoäthyl)]-guanylhydrazon ;

aus B und 1-(2-N-Methylpiperazinoäthyl)-aminoguanidin
B-1-[1-(2-N-Methylpiperazinoäthyl)]-guanylhydrazon ;

f) 1. aus 3-(4-Trifluormethylphenyl)-7-chlor-10-hydroxy-3,4-dihydro-acridin-1,9-(2H, 10H)-dion = A und Dimethylaminoessigsäure-hydrazid
A-1-(N-Dimethylaminoessigsäure)-hydrazon ;

aus A und N-Diäthylaminoessigsäurehydrazid
A-1-(N-Diäthylaminoessigsäure)-hydrazon Fp. 248° ;

aus A und Morpholinoessigsäurehydrazid
A-1-(Morpholinoessigsäure)-hydrazon ;

aus A und N-Methylpiperazino-N'-essigsäurehydrazid
A-1-(N-Methylpiperazino-N'-essigsäure)-hydrazon ;

aus A und 2-N-Dimethylaminopropionsäurehydrazid
A-1-(2-N-Dimethylaminopropionsäure)-hydrazon ;

aus A und 2-N-Diäthylaminopropionsäurehydrazid
A-1-(2-N-Diäthylaminopropionsäure)-hydrazon ;

aus A und 2-Morpholinopropionsäurehydrazid
A-1-(2-Morpholinopropionsäure)-hydrazon ;

aus A und 2-N-Methylpiperazino-N'-propionsäurehydrazid
A-1-(2-N-methylpiperazino-N'-propionsäure)-hydrazon ;

aus A und N-Trimethylammoniumessigsäurehydrazid-Chlorid
A-1-(N-Trimethylammoniumessigsäure)-hydrazon-Chlorid Fp. 244° ;

f) 2. aus 3-(4-Chlorphenyl)-7-chlor-10-hydroxy-3,4-dihydro-acridin-1,9-(2H, 10H)-dion = B und N-Dimethylaminoessigsäurehydrazid
B-1-(N-Dimethylaminoessigsäure)-hydrazon ;

aus B und N-Diäthylaminoessigsäurehydrazid
B-1-(N-Diäthylaminoessigsäure)-hydrazon Fp. 262° ;

aus B und Morpholinoessigsäurehydrazid
B-1-(Morpholinoessigsäure)-hydrazon ;

aus B und N-Methylpiperazino-N'-essigsäurehydrazid
B-1-(N-Methylpiperazino-N'-essigsäure)-hydrazon ;

aus B und 2-N-Dimethylaminopropionsäurehydrazid
B-1-(2-N-Dimethylaminopropionsäure)-hydrazon ;

aus B und 2-N-Diäthylaminopropionsäurehydrazid
B-1-(2-N-Diäthylaminopropionsäure)-hydrazon ;

aus B und 2-Morpholinopropionsäurehydrazid
B-1-(2-Morpholinopropionsäure)-hydrazon ;

aus B und 2-N-Methylpiperazino-N'-propionsäurehydrazid
B-1-(2-N-Methylpiperazino-N'-propionsäure)-hydrazon ;

aus B und N-Trimethylammoniumessigsäurehydrazid-Chlorid
B-1-(N-Trimethylammoniumessigsäure)-hydrazon-Chlorid Fp. 233° ;

g) 1. aux 3-(4-Trifluormethylphenyl)-7-chlor-10-hydroxy-3,4-dihydro-acridin-1,9-(2H,10H)-dion = A und Hydrazinoameisensäure-2-N-dimethylaminoäthylester
A-1-(2-N-Dimethylaminoäthyloxycarbonyl)-hydrazon ;

aus A und Hydrazinoameisensäure-2-N-diäthylaminoäthylester
A-1-(2-N-Diäthylaminoäthyloxycarbonyl)-hydrazon HCl Fp. 300° ;

aus A und Hydrazinoameisensäure-2-morpholinoäthylester
A-1-(2-Morpholinoäthyloxycarbonyl)-hydrazon ;

aus A und Hydrazinoameisensäure-2-N-methylpiperazino-N'-äthylester
A-1-(2-N-Methylpiperazino-N'-äthyloxycarbonyl)-hydrazon ;

g) 2. aus 3-(4-Trifluormethylphenyl)-7-chlor-10-hydroxy-3,4-dihydro-acridin-1,9-(2H,10H)-dion = B und Hydrazinoameisensäure-2-N-dimethylaminoäthylester
B-1-(2-N-Dimethylaminoäthyloxycarbonyl)-hydrazon ;

aus B und Hydrazinoameisensäure-2-N-diäthylaminoäthylester
B-1-(2-N-Diäthylaminoäthyloxycarbonyl)-hydrazon ;

aus B und Hydrazinoameisensäure-2-morpholinoäthylester
B-1-(2-Morpholinoäthyloxycarbonyl)-hydrazon ;

aus B und Hydrazinoameisensäure-2-N-methylpiperazino-N'-äthylester
B-1-(2-N-Methylpiperazino-N'-äthyloxycarbonyl)-hydrazon ;

B. Herstellungsbeispiele : ($R^6$ = H)

Beispiel 1

3-(4-Trifluormethylphenyl)-7-chlor-3,4-dihydroacridin-1,9-(2H,10H)-dion-1-(N-methyl-N'-piperazin)-imin

39,15 g (0,1 Mol) 3-(4-Tribluormethylphenyl)-7-chlor-3,4-dihydro-acridin-1,9-(2H,10H)-dion werden in 500 ml Äthanol suspendiert, 11,5 (0,1 Mol) N-Methyl-N'-aminopiperazin werden zugefügt und die Reaktionsmischung wird 90 Minuten zum Rückfluß erhitzt. Dabei bildet sich eine klare Lösung. Nach dem Abkühlen wird am Rotationsverdampfer das Äthanol abdestilliert und der feste Rückstand aus Toluol-Cyclohexan (1 : 1) umkristallisiert. Man erhält so 44 g = 90 % der Theorie der Titelverbindung in Form von hellgelben Kristallen mit dem Fp. 246 °C.

Die Herstellung des Ausgangsstoffes 3-(4-Trifluormethylphenyl)-7-chlor-3,4-dihydro-acridin-1,9-(2H,10H)-dion vom Fp. 385 °C erfolgt durch Umsetzung von 3-(4-Trifluormethyl-phenyl)-7-chlor-10-hydroxy-3,4-dihydro-acridin-1,9-(2H,10H)-dion vom Fp. 330° mit Phosphortrichlorid in Chloroform

(Dampf bad) analog der in DE-PS 2.337.474 angegebenen Vorschrift.

In analoger Weise wie in Beispiel 1 beschrieben werden folgende Verbindungen hergestellt :

a) 1. aus 3-(4-Trifluormethylphenyl)-7-chlor-3,4-dihydroacridin-1,9-(2H,10H)-dion = A (Herstellung vgl. DE-PS 2.337.474) und O-(2-N-Dimethylaminoäthyl)-hydroxylamin erhält man A-1-[O-(2-N-Dimethylaminoäthyl)-oxim] Fp. 270°

aus A und O-(3-N-Dimethylaminopropyl)-hydroxylamin
A-1-[O-(3-N-Dimethylaminopropyl)-oxim]

aus A und O-(2-N-Diäthylaminoäthyl)-hydroxylamin
A-1-[O-(2-N-Diäthylaminoäthyl)-oxim]

aus A und O-(3-N-Diäthylaminopropyl)-hydroxylamin
A-1-[O-(3-N-Diäthylaminopropyl)-oxim]

aus A und O-(2-Pyrrolidinoäthyl)-hydroxylamin
A-1-[O-(2-Pyrrolidinoäthyl)-oxim]

aus A und O-(2-Piperidinoäthyl)-hydroxylamin
A-1-[O-(2-Piperidinoäthyl)-oxim]

aus A und O-(2-Morpholinoäthyl)-hydroxylamin
A-1-[O-(2-Morpholinoäthyl)-oxim]

aus A und O-(2-Thiomorpholinoäthyl)-hydroxylamin
A-1-[O-(2-Thiomorpholinoäthyl)-oxim]

aus A und O-(2-N'-Methylpiperazinoäthyl)-hydroxylamin
A-1-[O-(2-N'-Methylpiperazinoäthyl)-oxim]

aus A und O-(2-N'-Benzylpiperazinoäthyl)-hydroxylamin
A-1-[O-(2-N'-Benzylpiperazinoäthyl)-oxim]

aus A und O-(2-N'-Phenylpiperazinoäthyl)-hydroxylamin
A-1-[O-(2-N'-Phenylpiperazinoäthyl)-oxim]

aus A und O-(3-N-Dimethylamino-2-hydroxypropyl)-hydroxylamin
A-1-[O-(3-N-Dimethylamino-2-hydroxypropyl)-oxim]

aus A und O-(3-N-Diäthylamino-2-hydroxypropyl)-hydroxylamin
A-1-[O-(3-N-Diäthylamino-2-hydroxypropyl)-oxim]

aus A und O-(3-Morpholino-2-hydroxypropyl)-hydroxylamin
A-1-[O-(3-Morpholino-2-hydroxypropyl)-oxim]

aus A und O-(3-N'-Methylpiperazino-2-hydroxypropyl)-hydroxylamin
A-1-[O-(3-N'-Methylpiperazino-2-hydroxypropyl)-oxim]

a) 2. aus 3-(4-Chlorphenyl)-7-chlor-3,4-dihydroacridin-1,9-(2H,10H)-dion = B (Herstellung nach DE-PS 2.337.474) und O-(2-N-Dimethylaminoäthyl)-hydroxylamin erhält man B-1-[O-(2-N-Dimethylamino-äthyl)-oxim] Fp. 287°

aus B und O-(3-N-Dimethylaminopropyl)-hydroxylamin
B-1-[O-(3-N-Dimethylaminopropyl)-oxim]

aus B und O-(2-N-Diäthylaminoäthyl)-hydroxylamin
B-1-[O-(2-N-Diäthylaminoäthyl)-oxim]

aus B und O-(3-N-Diäthylaminopropyl)-hydroxylamin
B-1-[O-(3-N-Diäthylaminopropyl)-oxim]

aus B und O-(2-Pyrrolidinoäthyl)-hydroxylamin
B-1-[O-(2-Pyrrolidinoäthyl)-oxim]

aus B und O-(2-Piperidinoäthyl)-hydroxylamin
B-1-[O-(2-Piperidinoäthyl)-oxim]

aus B und O-(2-Morpholinoäthyl)-hydroxylamin
B-1-[O-(2-Morpholinoäthyl)-oxim]

aus B und O-(2-Thiomorpholinoäthyl)-hydroxylamin
B-1-[O-(2-Thiomorpholinoäthyl)-oxim]

aus B und O-(2-N'-Methylpiperazinoäthyl)-hydroxylamin
B-1-[O-(2-N'-Methylpiperazinoäthyl)-oxim]

aus B und O-(2-N'-Benzylpiperazinoäthyl)-hydroxylamin
B-1-[O-(2-N'-Benzylpiperazinoäthyl)-oxim]

aus B und O-(2-N'-Phenylpiperazinoäthyl)-hydroxylamin
B-1-[O-(2-N'-Phenylpiperazinoäthyl)-oxim]

aus B und O-(3-N-Dimethylamino-2-hydroxypropyl)-hydroxylamin
B-1-[O-(3-N-Dimethylamino-2-hydroxypropyl)-oxim]

aus B und O-(3-N-Diäthylamino-2-hydroxypropyl)-hydroxylamin
B-1-[O-(3-N-Diäthylamino-2-hydroxypropyl)-oxim

aus B und O-(3-Morpholino-2-hydroxypropyl)-hydroxylamin
B-1-[O-(3-Morpholino-2-hydroxypropyl)-oxim]

aus B und O-(3-N'-Methylpiperazino-2-hydroxypropyl)-hydroxylamin
B-1-[O-(3-N'-Methylpiperazino-2-hydroxypropyl)-oxim]

b) 1. aus 3-(4-Trifluormethylphenyl)-7-chlor-3,4-dihydroacridin-1,9-(2H,10H)-dion = A und N,N-Dimethylhydrazin
A-1-N,N-Dimethylhydrazon Fp. 240°

aus A und N,N-Di-butylhydrazin
A-1-N,N-Di-n-butylhydrazon ;

aus A und N-Aminopyrrolidin
A-1-N-Pyrrolidinimin ;

aus A und N-Aminomorpholin
A-1-N-Morpholinimin ;

aus A und N-2-Hydroxyäthyl-N'-aminopiperazin
A-1-N-2-Hydroxyäthyl-N'-piperazinimin Fp. 245° ;

aus A und N-Benzyl-N'-aminopiperazin
A-1-N-Benzyl-N'-piperazinimin ;

aus A und N-Phenyl-N'-aminopiperazin
A-1-N-Phenyl-N'-piperazinimin ;

b) 2. aus 3-(4-Chlorphenyl)-7-chlor-3,4-dihydro-acridin-1,9-(2H,10H)-dion = B und N,N-Dimethylhydrazin
B-1-N,N-Dimethylhydrazon ;

aus B und N,N-Di-n-butylhydrazin
B-1-N,N-butylhydrazon ;

aus B und N-Aminopyrrolidin
B-1-N-Pyrrolidinimin ;

aus B und N-Aminomorpholin
B-1-N-Morpholinimin ;

aus B und N-Methyl-N'-aminopiperazin
B-1-N-Methyl-N'-piperazinimin Fp. 270° ;

aus B und N-2-Hydroxyäthyl-N'-aminopiperazin
B-1-N-2-Hydroxyäthyl-N'-piperazinimin Fp 190° ;

aus B und N-Benzyl-N'-aminopiperazin
B-1-N-Benzyl-N'-piperazinimin ;

aus B und N-Phenyl-N'-aminopiperazin
B-1-N-Phenyl-N'-piperazinimin ;

b) 3. aus 3-Phenyl-7-chlor-3,4-dihydro-acridin-1,9-(2H,10H)-dion = D und N-Methyl-N'-aminopiperazin
D-1-N-Methyl-N'-piperazinimin ;

4. aus 3-(4-Fluorphenyl)-7-chlor-3,4-dihydro-acridin-1,9-(2H,10H)-dion = E und N-Methyl-N'-aminopiperazin
E-1-N-Methyl-N'-piperazinimin ;

5. aus 3-(2-Chlorphenyl)-7-chlor-3,4-dihydro-acridin-1,9-(2H,10H)-dion = G und N-Methyl-N'-aminopiperazin
G-1-N-Methyl-N'-piperazinimin ;

6. aus 3-(3-Chlorphenyl)-7-chlor-3,4-dihydro-acridin-1,9-(2H,10H)-dion = L und N-Methyl-N'-aminopiperazin
L-1-N-Methyl-N'-piperazinimin ;

7. aus 3-(2,4-Dichlorphenyl)-7-chlor-3,4-dihydro-acridin-1,9-(2H,10H)-dion = M und N-Methyl-N'-aminopiperazin
M-1-N-Methyl-N'-piperazinimin Fp. 195° ;

8. aus 3-(3,4-Dichlorphenyl)-7-chlor-3,4-dihydro-acridin-1,9-(2H,10H)-dion = Q und N-Methyl-N'-aminopiperazin
Q-1-N-Methyl-N'-piperazinimin ;

9. aus 3-(2-Trifluormethylphenyl)-7-chlor-3,4-dihydro-acridin-1,9-(2H,10H)-dion = T und N-Methyl-N'-aminopiperazin
T-1-N-Methyl-N'-piperazinimin ;

10. aus 3-(2-chlor-4-trifluormethylphenyl)-7-chlor-10-hydroxy-3,4-dihydro-acridin-1,9-(2H,10H)-dion = U und N-Methyl-N'-aminopiperazin
U-1-N-Methyl-N'-piperazinimin Fp. 242° ;

11. aus 3-(4-Methoxyphenyl)-7-chlor-3,4-dihydro-acridin-1,9-(2H,10H)-dion = V und N-Methyl-N'-aminopiperazin
V-1-N-Methyl-N'-piperazinimin ;

12. aus 3-(4-Nitrophenyl)-7-chlor-3,4-dihydro-acridin-1,9-(2H,10H)-dion = W und N-Methyl-N'-aminopiperazin
W-1-N-Methyl-N'-piperazinimin ;

c) 1. aus A und 2-N-Dimethylaminoäthylhydrazin
A-1-2-N-Dimethylaminoäthylhydrazon ;

aus A und 2-N-Diäthylaminoäthylhydrazin
A-1-2-N-Diäthylaminoäthylhydrazon Fp. 217° ;

aus A und 2-Piperidinoäthylhydrazin
A-1-(2-Piperidinoäthyl)hydrazon ;

aus A und 2-N-Methylpiperazinoäthylhydrazin
A-1-(2-N-Methylpiperazinoäthyl)hydrazon ;

c) 2. aus B und 2-N-Dimethylaminoäthylhydrazin

B-1-2-N-Dimethylaminoäthylhydrazon ;

aus B und 2-N-Diäthylaminoäthylhydrazin
B-1-2-N-Diäthylaminoäthylhydrazon ;

aus B und 2-Piperidinoäthylhydrazin
B-1-(2-Piperidinoäthyl)hydrazon ;

aus B und 2-N-Methylpiperazinoäthylhydrazin
B-1-(2-N-Methylpiperazinoäthyl)hydrazon ;

d) 1. aus 3-(4-Trifluormethylphenyl)-7-chlor-3,4-dihydro-acridin-1,9-(2H,10H)-dion = A und 1,1-Dimethyl-semicarbazid
A-1-(1,1-Dimethyl)-semicarbazon ;

aus A und 1,1-Dimethyl-thiosemicarbazid
A-1-(1,1-Dimethyl)-thiosemicarbazon ;

aus A und 1,1-Dimethyl-aminoguanidin
A-1-(1,1-Dimethyl)-guanylhydrazon ;

aus A und N-Hydrazinocarbonyl-N'-methylpiperazin
A-1-(N'-Methylpiperazino-N-carbonyl)-hydrazon ;

aus A und N-Hydrazinothiocarbonyl-N'-methylpiperazin
A-1-(N'-Methylpiperazino-N-thiocarbonyl)-hydrazon ;

aus A und N-Hydrazinocarbimido-N'-methylpiperazin
A-1-(N'-Methylpiperazino-N-carbimido)-hydrazon ;

d) 2. aus 3-(4-Chlorphenyl)-7-chlor-3,4-dihydro-acridin-1,9-(2H,10H)-dion = B und 1,1-Dimethyl-semicarbazid
B-1-(1,1-Dimethyl)-semicarbazon ;

aus B und 1,1-Dimethyl-thiosemicarbazid
B-1-(1,1-Dimethyl)-thiosemicarbazon ;

aus B und 1,1-Dimethyl-aminoguanidin
B-1-(1,1-Dimethyl)-guanylhydrazon ;

aus B und N-Hydrazinocarbonyl-N'-methylpiperazin
B-1-(N'-Methylpiperazino-N-carbonyl)-hydrazon ;

aus B und N-Hydrazinothiocarbonyl-N'-methylpiperazin
B-1-(N'-Methylpiperazino-N-thiocarbonyl)-hydrazon ;

aus B und N-Hydrazinothiocarbimido-N'-methylpiperazin
B-1-(N'-Methylpiperazino-N-carbimido)-hydrazon ;

e) 1. aus A und 1-(2-N-Dimethylaminoäthyl)-semicarbazid
A-1-[1-(2-N-Dimethylaminoäthyl)]-semicarbazon ;

aus A und 1-(2-N-Diäthylaminoäthyl)-semicarbazid
A-1-[1-(2-N-Diäthylaminoäthyl)]-semicarbazon ;

aus A und 1-(2-N-Methylpiperazinoäthyl)-semicarbazid
A-1-[1-(2-N-Methylpiperazinoäthyl)]-semicarbazon ;

aus A und 1-(2-N-Dimethylaminoäthyl)-thiosemicarbazid
A-1-[1-(2-N-Dimethylaminoäthyl)]-thiosemicarbazon ;

aus A und 1-(2-N-Diäthylaminoäthyl)-thiosemicarbazid
A-1-[1-(2-N-Diäthylaminoäthyl)]-thiosemicarbazon ;

aus A und 1-(2-N-Methylpiperazinoäthyl)-thiosemicarbazid

A-1-[1-(2-N-Methylpiperazinoäthyl)]-thiosemicarbazon ;

aus A und 1-(2-N-DImethylaminoäthyl)-aminoguanidin
A-1-[1-(2-N-Dimethylaminoäthyl)]-guanylhydrazon ;

aus A und 1-(2-N-Diäthylaminoäthyl)-aminoguanidin
A-1-[1-(2-N-Diäthylaminoäthyl)]-guanylhydrazon ;

aus A und 1-(2-N-Methylpiperazinoäthyl)-aminoguanidin
A-1-[1-(2-N-Methylpiperazinoäthyl)]-guanylhydrazon ;

e) 2. aus B und 1-(2-N-Dimethylaminoäthyl)-semicarbazid
B-1-[1-(2-N-Dimethylaminoäthyl)]-semicarbazon ;

aus B und 1-(2-N-Diäthylaminoäthyl)-semicarbazid
B-1-[1-(2-N-Diäthylaminoäthyl)]-semicarbazon ;

aus B und 1-(2-N-Methylpiperazinoäthyl)-semicarbazid
B-1-[1-(2-N-Methylpiperazinoäthyl)]-semicarbazon ;

aus B und 1-(2-N-Dimethylaminoäthyl)-thiosemicarbazid
B-1-[1-(2-N-Dimethylaminoäthyl)]-thiosemicarbazon ;

aus B und 1-(2-N-Diäthylaminoäthyl)-thiosemicarbazid
B-1-[1-(2-N-Diathylaminoäthyl)]-thiosemicarbazon ;

aus B und 1-(2-N-Methylpiperazinoäthyl)-thiosemicarbazid
B-1-[1-(2-N-Methylpiperazinoäthyl)]-thiosemicarbazon ;

aus B und 1-(2-N-Dimethylaminoäthyl)-aminoguanidin
B-1-[1-(2-N-Dimethylaminoäthyl)]-guanylhydrazon ;

aus B und 1-(2-N-Diäthylaminoäthyl)-aminoguanidin
B-1-[1-(2-N-Diäthylaminoathyl)]-guanylhydrazon ;

aus B und 1-(2-N-Methylpiperazinoäthyl)-aminoguanidin
B-1-[1-(2-N-Methylpiperazinoäthyl)]-guanylhydrazon ;

f) 1. aus 3-(4-Trifluormethylphenyl)-7-chlor-3,4-dihydro-acridin-1,9-(2H,10H)-dion = A und Dimethylaminoessigsäure-hydrazid
A-1-(N-Dimethylaminoessigsäure)-hydrazon ;

aus A und N-Diäthylaminoessigsäurehydrazid
A-1-(N-Diäthylaminoessigsäure)-hydrazon ;

aus A und Morpholinoessigsäurehydrazid
A-1-(Morpholinoessigsäure)-hydrazon ;

aus A und N-Methylpiperazino-N'-essigsäurehydrazid
A-1-(N-Methylpiperazino-N'-essigsäure)-hydrazon ;

aus A und 2-N-Dimethylaminopropionsäurehydrazid
A-1-(2-N-Dimethylaminopropionsäure)-hydrazon ;

aus A und 2-N-Diäthylaminopropionsäurehydrazid
A-1-(2-N-Diäthylaminopropionsäure)-hydrazon ;

aus A und 2-Morpholinopropionsäurehydrazid
A-1-(2-Morpholinopropionsäure)-hydrazon ;

aus A und 2-N-Methylpiperazino-N'-propionsäurehydrazid
A-1-(2-N-Methylpiperazino)N'-propionsäure)-hydrazon ;

aus A und N-Trimethylammoniumessigsäurehydrazid-Chlorid
A-1-(N-Trimethylammoniumessigsäure)-hydrazon-Chlorid Fp. 296° ;

f) 2. aus 3-(4-Chlorphenyl)-7-chlor-3,4-dihydro-acridin-1,9-(2H,10H)-dion = B und Dimethylaminoessigsäurehydrazid
B-1-(N-Dimethylaminoessigsäure)-hydrazon ;

aus B und N-Diäthylaminoessigsäurehydrazid
B-1-(N-Diäthylaminoessigsäure)-hydrazon ;

aus B und Morpholinoessigsäurehydrazid
B-1-(Morpholinoessigsäure)-hydrazon ;

aus B und N-Methylpiperazino-N'-essigsäurehydrazid
B-1-(N-Methylpiperazino-N'-essigsäure)-hydrazon ;

aus B und 2-N-Dimethylaminopropionsäurehydrazid
B-1-(2-N-Dimethylaminopropionsäure)-hydrazon ;

aus B und 2-N-Diäthylaminopropionsäurehydrazid
B-1-(2-N-Diäthylaminopropionsäure)-hydrazon ;

aus B und 2-Morpholinopropionsäurehydrazid
B-1-(2-Morpholinopropionsäure)-hydrazon ;

aus B und 2-N-Methylpiperazino-N'-propionsäurehydrazid
B-1-(2-N-Methylpiperazino-N'-propionsäure)-hydrazon ;

aus B und N-Trimethylammoniumessigsäurehydrazid-Chlorid
B-1-(N-Trimethylammoniumessigsäure)-hydrazon-Chlorid Fp. 280° ;

g) 1. aus 3-(4-Trifluormethylphenyl)-7-chlor-3,4-dihydro-acridin-1,9-(2H,10H)-dion = A und Hydrazinoameisensäure-2-N-dimethylaminoäthylester
A-1-(2-N-Dimethylaminoäthyloxycarbonyl)-hydrazon ;

aus A und Hydrazinoameisensäure-2-N-diäthylaminoäthylester
A-1-(2-N-Diäthylaminoäthyloxycarbonyl)-hydrazon HCl ;

aus A und Hydrazinoameisensäure-2-morpholinoäthylester
A-1-(2-Morpholinoäthyloxycarbonyl)-hydrazon ;

aus A und Hydrazinomeisensäure-2-N-methylpiperazino-N'-äthylester
A-1-(2-N-Methylpiperazino-N'-äthyloxycarbonyl)-hydrazon ;

g) 2. aus 3-(4-Trifluormethylphenyl)-7-chlor-3,4-dihydro-acridin-1,9-(2H,10H)-dion = B und Hydrazinoameisensäure-2-N-dimethylaminoäthylester
B-1-(2-N-Dimethylaminoäthyloxycarbonyl)-hydrazon ;

aus B und Hydrazinoameisensäure-2-N-diäthylaminoäthylester
B-1-(2-N-Diäthylaminoäthyloxycarbonyl)-hydrazon ;

aus B und Hydrazinoameisensäure-2-morpholinoäthylester
B-1-(2-Morpholinoäthyloxycarbonyl)-hydrazon ;

aus B und Hydrazinoameisensäure-2-N-methylpiperazino-N'-äthylester
B-1-(2-N-Methylpiperazino-N'-äthyloxycarbonyl)-hydrazon.

**Patentansprüche** (für die Vertragsstaaten : BE, CH, DE, FR, GB, IT, LI, LU, NL, SE)

1. 3-Aryl-7-chlor-3,4-dihydro-acridin-1,9-(2H-10H)-dion-1-oxime und -1-hydrazonderivate der Formel I

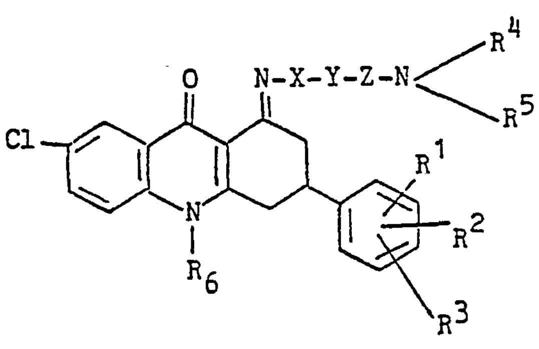

(I)

worin $R^1$, $R^2$ und $R^3$ gleich oder verschieden sind und Wasserstoff, Halogen, $C_1$-$C_4$-Alkyl, Trifluormethyl, $C_1$-$C_4$-Alkoxy, Trifluormethoxy, Difluorchlormethoxy, 1,1,2,2-Tetrafluoräthoxy, Phenoxy, Halogenphenoxy, $C_1$-$C_4$-Alkyl-, Phenyl- und Naphthyl-thio, $C_1$-$C_4$-Alkyl-, Phenyl- und Naphthyl-sulfinyl, $C_1$-$C_4$-Alkyl-, Phenyl- und Naphthyl-sulfonyl, Trifluormethylthio, Carbamyl, Sulfamyl, Cyano oder Nitro bedeuten,

a) X Sauerstoff,

Y eine einfache Bindung,

Z eine gerade oder verzweigte Alkylenkette mit 2 bis 5 Kohlenstoffatomen, die gegebenenfalls mit einer Hydroxygruppe substituiert ist, oder

b) X, Y und Z eine einfache Bindung, oder

c) X die —NR-Gruppe, worin R Wasserstoff oder $C_1$-$C_4$-Alkyl bedeutet,

Y eine einfache Bindung,

Z eine gerade oder verzweigte Alkylenkette mit 2 bis 5 Kohlenstoffatomen, oder

d) X die —NR-Gruppe, worin R Wasserstoff oder $C_1$-$C_4$-Alkyl bedeutet,

Y die Carbonyl-, Thiocarbonyl- oder Iminocarbonyl-Gruppe,

Z eine einfache Bindung, oder

e) X die —NR-Gruppe, worin R Wasserstoff oder $C_1$-$C_4$-Alkyl bedeutet,

Y die Carbonamid-, Thiocarbonamid- oder Carbonamidin-Gruppe,

Z eine gerade oder verzweigte Alkylenkette mit 2 bis 5 Kohlenstoffatomen, oder

f) X die —NR-Gruppe, worin R Wasserstoff oder $C_1$-$C_4$-Alkyl bedeutet,

Y die Carbonylgruppe,

Z eine gerade oder verzweigte Alkylenkette mit 1 bis 3 Kohlenstoffatomen, oder

g) X die —NR-Gruppe, worin R Wasserstoff oder $C_1$-$C_4$-Alkyl bedeutet,

Y die Carboxygruppe,

Z eine gerade oder verzweigte Alkylenkette mit 2 bis 5 Kohlenstoffatomen bedeuten und

$R^4$ und $R^5$ gleich oder verschieden sind und Wasserstoff oder geradkettiges oder verzweigtes $C_1$-$C_4$-Alkyl bedeuten, wobei $R^4$ und $R^5$ nicht gleichzeitig Wasserstoff sind, oder $R^4$ und $R^5$ sind zusammen eine Alkylenkette mit 4 bis 6 Kohlenstoffatomen, die mit dem Stickstoffatom einen 5- bis 7-gliedrigen heterocyclischen Ring bilden, worin eine $-CH_2$-Gruppe durch ein weiteres Heteroatom aus der Gruppe Stickstoff, Sauerstoff oder Schwefel ersetzt sein kann, und wobei der heterocyclische Ring seinerseits substituiert sein kann mit einer, gegebenenfalls mit einer Hydroxygruppe substituierten, Alkylgruppe mit einem bis drei Kohlenstoffatomen oder mit Phenyl- oder Naphthyl-$C_1$-$C_3$-Alkyl oder Phenyl oder Naphthyl, gegebenenfalls substituiert mit Methyl, Methoxy, Chlor oder Trifluormethyl, und

$R^6$ Wasserstoff oder die Hydroxylgruppe bedeutet, sowie deren physiologisch verträgliche Säureadditions- und Ammoniumsalze.

2. Eine Verbindung gemäß Anspruch 1, dadurch gekennzeichnet, daß $R^4$ und $R^5$ von Wasserstoff verschieden sind.

3. Eine Verbindung gemäß Anspruch 2, dadurch gekennzeichnet, daß $R^1$ und $R^2$ Wasserstoff, $R^3$ Chlor oder Trifluormethyl, X, Y und Z eine einfache Bindung und $R^4$ und $R^5$ zusammen mit dem Stickstoffatom einen Piperazinring, der gegebenenfalls am Stickstoff substituiert ist, bedeuten.

4. Eine Verbindung gemäß Anspruch 2, dadurch gekennzeichnet, daß sie 3-(4-Trifluormethylphenyl)-7-chlor-3,4-dihydro-acridin-1,9-(2H,10H)-dion-1-(N-methyl-N'-piperazin)-imin bzw. 1-N-2-hydroxyäthyl-N'-piperazinimin ist.

5. Eine Verbindung gemäß Anspruch 2, dadurch gekennzeichnet, daß sie 3-(4-Chlorphenyl)-7-chlor-3,4-dihydro-acridin-1,9-(2H,20H)-dion-1-N-methyl-N'-piperazinimin bzw. -1-N-2-hydroxyäthyl-N'-piperazinimin ist.

6. Verfahren zur Herstellung von 3-Aryl-7-chlor-3,4-dihydroacridin-1,9-(2H,10H)-dion-1-oximen und -1-hydrazonderivaten gemäß einem oder mehreren der Ansprüche 1 bis 5, dadurch gekennzeichnet, daß man 3-Aryl-7-chlor-3,4-dihydro-acridin-1,9-(2H,10H)-dion der Formel II

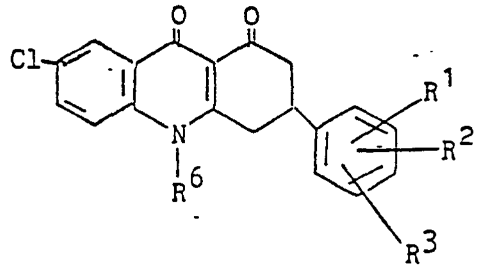

(II)

worin $R^1$, $R^2$, $R^3$ und $R^6$ die zu Formel I angegebene Bedeutung haben, mit einer Verbindung der Formel III

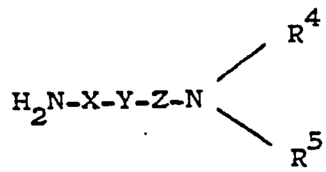

(III)

worin X, Y, Z sowie $R^4$ und $R^5$ die zu Formel I angegebene Bedeutung haben, oder mit deren Salzen umsetzt, und das Reaktionsprodukt gegebenenfalls mit einer physiologisch vertraglichen Säure in ein Additionssalz oder mit einem Alkylierungsmittel in ein Ammoniumsalz überführt.

7. Pharmazeutisches Präparat enthaltend eine Verbindung gemäß einem oder mehreren der Ansprüche 1 bis 5.

8. Verfahren zur Herstellung eines pharmazeutischen Präparates gemäß Anspruch 7, dadurch gekennzeichnet, daß man eine Verbindung gemäß einem oder mehreren der Ansprüche 1 bis 5 in eine geeignete Applikationsform bringt.

9. Verbindung gemäß einem oder mehreren der Ansprüche 1 bis 5 bzw. pharmazeutisches Präparat gemäß Anspruch 7 zur Bekämpfung von Infektionen durch Protozoen bei Mensch und Tier.

**Patentanspruch** (für den Vertragsstaat AT)

1. Verfahren zur Herstellung von 3-Aryl-7-chlor-3,4-dihydroacridin-1,9-(2H,10H)-dion-1-oximen und -1-hydrazonderivaten der Formel I

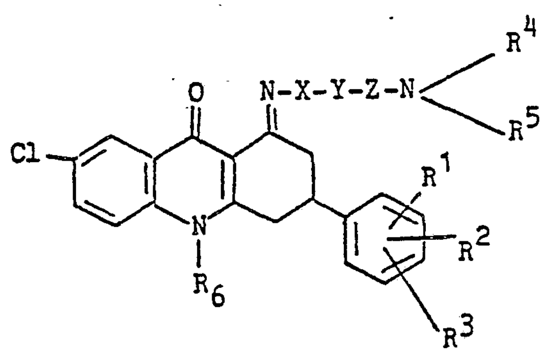

(I)

worin $R^1$, $R^2$ und $R^3$ gleich oder verschieden sind und Wasserstoff, Halogen, $C_1$-$C_4$-Alkyl, Trifluormethyl, $C_1$-$C_4$-Alkoxy, Trifluormethoxy, Difluorchlormethoxy, 1,1,2,2-Tetrafluoräthoxy, Phenoxy, Halogenphenoxy, $C_1$-$C_4$-Alkyl-, Phenyl- und Naphthyl-thio, $C_1$-$C_4$-Alkyl-, Phenyl- und Naphthyl-sulfinyl, $C_1$-$C_4$-Alkyl-, Phenyl- und Naphthyl-sulfonyl, Trifluormethylthio, Carbamyl, Sulfamyl, Cyano oder Nitro bedeuten,

a) X Sauerstoff,
Y eine einfache Bindung,
Z eine gerade oder verzweigte Alkylenkette mit 2 bis 5 Kohlenstoffatomen, die gegebenenfalls mit einer Hydroxygruppe substituiert ist, oder

b) X, Y und Z eine einfache Bindung, oder

c) X die —NR-Gruppe, worin R Wasserstoff oder $C_1$-$C_4$-Alkyl bedeutet,
Y eine einfache Bindung,
Z eine gerade oder verzweigte Alkylenkette mit 2 bis 5 Kohlenstoffatomen, oder

d) X die —NR-Gruppe, worin R Wasserstoff oder $C_1$-$C_4$-Alkyl bedeutet,
Y die Carbonyl-, Thiocarbonyl- oder Iminocarbonyl-Gruppe,
Z eine einfach Bindung, oder

e) X die —NR-Gruppe, worin R Wasserstoff oder $C_1$-$C_4$-Alkyl bedeutet,
Y die Carbonamid-, Thiocarbonamid- oder Carbonamidin-Gruppe,
Z eine gerade oder verzweigte Alkylenkette mit 2 bis 5 Kohlenstoffatomen, oder

f) X die —NR-Gruppe, worin R Wasserstoff oder $C_1$-$C_4$-Alkyl bedeutet,
Y die Carbonylgruppe,
Z eine gerade oder verzweigte Alkylenkette mit 1 bis 3 Kohlenstoffatomen, oder

g) X die —NR-Gruppe, worin R Wasserstoff oder $C_1$-$C_4$-Alkyl bedeutet,

Y die Carboxygruppe,

Z eine gerade oder verzweigte Alkylenkette mit 2 bis 5 Kohlenstoffatomen bedeuten und

$R^4$ und $R^5$ gleich oder verschieden sind und Wasserstoff oder geradkettiges oder verzweigtes $C_1$-$C_4$-Alkyl bedeuten wobei $R^4$ und $R^5$ nicht gleichzeitig Wasserstoff sind, oder $R^4$ und $R^5$ sind zusammen eine Alkylenkette mit 4 bis 6 Kohlenstoffatomen, die mit dem Stickstoffatom einen 5- bis 7-gliedrigen heterocyclischen Ring bilden, worin eine —$CH_2$-Gruppe durch ein weiteres Heteroatom aus der Gruppe Stickstoff, Sauerstoff oder Schwefel ersetzt sein kann, und wobei der heterocyclische Ring seinerseits substituiert sein kann mit einer, gegebenenfalls mit einer Hydroxygruppe substituierten, Alkylgruppe mit einem bis drei Kohlenstoffatomen oder mit Phenyl- oder Naphthyl, $C_1$-$C_3$-Alkyl oder Phenyl oder Naphthyl, gegebenenfalls substituiert mit Methyl, Methoxy, Chlor oder Trifluormethyl, und

$R^6$ Wasserstoff oder die Hydroxylgruppe bedeutet, sowie deren physiologisch verträglichen Säureadditions- und Ammoniumsalzen, dadurch gekennzeichnet, daß man 3-Aryl-7-Chlor-3,4-dihydro-acridin-1,9-(2H, 10H)-dion der Formel II

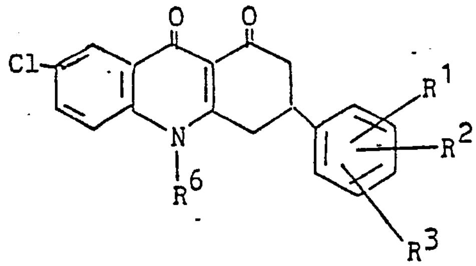

(II)

worin $R^1$, $R^2$, $R^3$ und $R^6$ die zu Formel I angegebene Bedeutung haben, mit einer Verbindung der Formel III

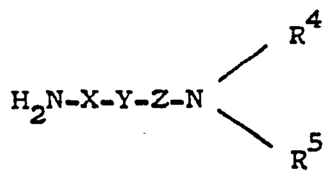

(III)

worin X, Y, Z sowie $R^4$ und $R^5$ die zu Formel I angegebene Bedeutung haben, oder mit deren Salzen umsetzt, und das Reaktionsprodukt gegebenenfalls mit einer physiologisch verträglichen Säure in ein Additionssalz oder mit einem Alkylierungsmittel in ein Ammoniumsalz überführt.

2. Verfahren nach Anspruch 1, dadurch gekennzeichnet, daß solche Verbindungen der Formel III umgesetzt werden, in denen $R^4$ und $R^5$ von Wasserstoff verschieden sind.

3. Verfahren nach Anspruch 2, dadurch gekennzeichnet, daß solche Verbindungen der Formel II, in denen $R^1$ und $R^2$ Wasserstoff und $R^3$ Chlor oder Trifluormethyl sind, mit solchen Verbindungen der Formel III umgesetzt werden, in denen X, Y und Z eine einfache Bindung und $R^4$ und $R^5$ zusammen mit dem Stickstoffatom einen Piperazinring bedeuten, der gegebenenfalls am Stickstoff substituiert ist.

4. Verfahren nach Anspruch 2, dadurch gekennzeichnet, daß das 3-(4-Trifluormethylphenyl)-7-chlor-3,4-dihydroacridin-1,9-(2H, 10H)-dion mit N-Methyl-N'-aminopiperazin bzw. N-2-Hydroxyäthyl-N'-aminopiperazin umgesetzt wird.

5. Verfahren nach Anspruch 2, dadurch gekennzeichnet, daß 3-(4-Chlorphenyl)-7-chlor-3,4-dihydroacridin-1,9-(2H, 10H)-dion mit N-Methyl-N'-aminopiperazin bzw. N-2-Hydroxyäthyl-N'-aminopiperazin umgesetzt wird.

**Claims** (for the Contracting States : BE, CH, DE, FR, GB, IT, LI, LU, NL, SE)

1. 3-Aryl-7-chloro-3,4-dihydroacridine-1,9-(2H, 10H)-dione 1-oximes and 1-hydrazone derivatives of the formula I

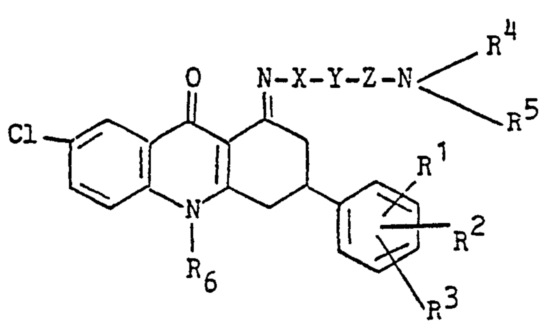

(I)

in which $R^1$, $R^2$ and $R^3$ are identical or different and denote hydrogen, halogen, $C_1$-$C_4$-alkyl, trifluoromethyl, $C_1$-$C_4$-alkoxy, trifluoromethoxy, difluorochloromethoxy, 1,1,2,2-tetrafluoroethoxy, phenoxy, halogenophenoxy, $C_1$-$C_4$-alkylthio, phenylthio and naphthylthio, $C_1$-$C_4$-alkylsulfinyl, phenylsulfinyl and naphthylsulfinyl, $C_1$-$C_4$-alkylsulfonyl, phenylsulfonyl and naphthylsulfonyl, trifluoromethylthio, carbamyl, sulfamyl, cyano or nitro,

a) X denotes oxygen,

Y denotes a single bond and

Z denotes a straight-chain or branched alkylene chain having 2 to 5 carbon atoms, which is optionally substituted with a hydroxy group, or

b) X, Y and Z denote a single bond, or

c) X denotes the —NR— group, in which R denotes hydrogen or $C_1$-$C_4$-alkyl,

Y denotes a single bond and

Z denote a straight-chain or branched alkylene chain having 2 to 5 carbon atoms, or

d) X denotes the —NR— group, in which R denotes hydrogen or $C_1$-$C_4$-alkyl,

Y denotes the carbonyl, thiocarbonyl or iminocarbonyl group and

Z denotes a single bond, or

e) X denotes the —NR— group, in which R denotes hydrogen or $C_1$-$C_4$-alkyl,

Y denotes the carboxamido, thiocarboxamido or carboxamidino group and

Z denotes a straight-chain or branched alkylene chain having 2 to 5 carbon atoms, or

f) X denotes the —NR— group, in which R denotes hydrogen or $C_1$-$C_4$-alkyl,

Y denotes the carbonyl group and

Z denotes a straight-chain or branched alkylene chain having 1 to 3 carbon atoms, or

g) X denotes the —NR— group, in which R denotes hydrogen or $C_1$-$C_4$-alkyl,

Y denotes the carbonyl group and

Z denotes a straight-chain or branched alkylene chain having 2 to 5 carbon atoms, and

$R^4$ and $R^5$ are identical or different and denote hydrogen or straight-chain or branched $C_1$-$C_4$-alkyl, whereby $R^4$ and

$R^5$ are not hydrogen at the same time, or $R^4$ and $R^5$ together are an alkylene chain having 4 to 6 carbon atoms which form, with the nitrogen atom, a 5- to 7-membered heterocyclic ring, in which it is possible for one $—CH_2—$ group to be replaced by another heteroatom from the group comprising nitrogen, oxygen or sulfur, and it being possible for the heterocyclic ring in turn to be substituted with an alkyl group having one to three carbon atoms which is optionally substituted with a hydroxyl group, or with phenyl-$C_1$-$C_3$-alkyl or naphthyl-$C_1$-$C_3$-alkyl or with phenyl or naphthyl, which is optionally substituted with methyl, methoxy, chlorine or trifluoromethyl, and

$R^6$ denotes hydrogen or the hydroxyl group, and their physiologically tolerated acid addition and ammonium salts.

2. A compound as claimed in claim 1, characterized in $R^4$ and $R^5$ being different from hydrogen.

3. A compound as claimed in claim 2, characterized in $R^1$ and $R^2$ denoting hydrogen, $R^3$ denoting chlorine or trifluoromethyl, X, Y and Z denoting a single bond and $R^4$ and $R^5$ denoting, together with the nitrogen atom, a piperazine ring which is optionally substituted on the nitrogen.

4. A compound as claimed in claim 2, characterized in being 3-(4-Trifluoromethylphenyl)-7-chloro-3,4-dihydro-acridine-1,9-(2H, 10H)-dione 1-(N-methyl-N'-piperazin)-imine or 1-N-2-hydroxyethyl-N'-piperazinimine respectively.

5. A compound as claimed in claim 2, characterized in being 3-(4-chlorophenyl)-7-chloro-3,4-dihydroacridine-1,9-(2H, 10H)-dione 1-N-methyl-N'-piperazinimine or 1-N-2-hydroxyethyl-N'-piperazinimine respectively.

6. A process for the preparation of 3-aryl-7-chloro-3,4-dihydroacridine-1,9-(2H, 10H)-dione 1-oximes and 1-hydrazone derivatives as claimed in one or more of claims 1-5, characterized in reacting 3-aryl-7-chloro-3,4-dihydroacridine-1,9-(2H, 10H)-dione of the formula II

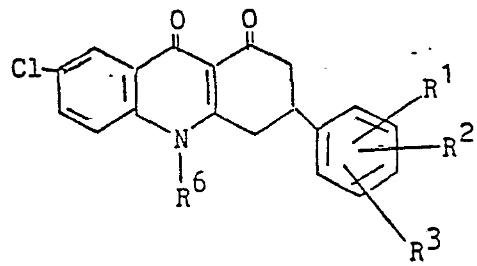

(II)

in which $R^1$, $R^2$, $R^3$ and $R^6$ have the meanings indicated for formula I, with a compound of the formula III

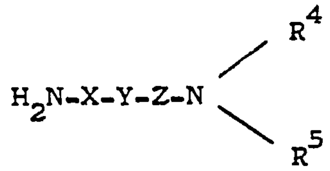

(III)

in which X, Y, Z and $R^4$ and $R^5$ have the meanings indicated for formula I, or with their salts, and converting the reaction product, where appropriate, with a physiologically tolerated acid into an addition salt or with an alkylating agent into an ammonium salt.

7. A pharmaceutical product containing a compound as claimed in one or more of claims 1-5.

8. A process for the preparation of a pharmaceutical product as claimed in claim 7, characterized in bringing a compound as claimed in one or more of claims 1-5 into a suitable form for administration.

9. A compound as claimed in one or more of claims 1-5 or a pharmaceutical product as claimed in claim 7, respectively, for combating infections caused by protozoae in men and animals.

**Claims** (for the Contracting State AT)

1. A process for the production of 3-Aryl-7-chloro-3,4-dihydroacridine-1,9-(2H, 10H)-dione 1-oximes and 1-hydrazone derivatives of the formula I

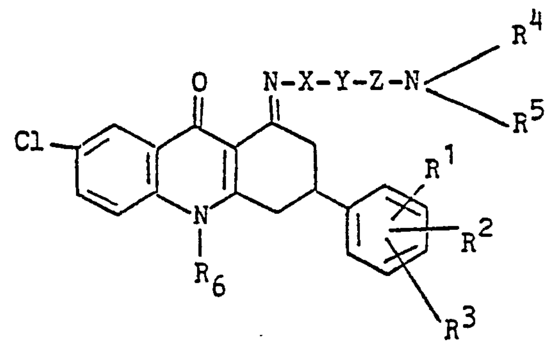

(I)

in which $R^1$, $R^2$ and $R^3$ are identical or different and denote hydrogen, halogen, $C_1$-$C_4$-alkyl, trifluoromethyl, $C_1$-$C_4$-alkoxy, trifluoromethoxy, difluorochloromethoxy, 1,1,2,2-tetrafluoroethoxy, phenoxy, halogenophenoxy, $C_1$-$C_4$-alkylthio, phenylthio and naphthylthio, $C_1$-$C_4$-alkylsulfinyl, phenylsulfinyl and naphthylsulfinyl, $C_1$-$C_4$-alkylsulfonyl, phenylsulfonyl and naphthylsulfonyl, trifluoromethylthio, carbamyl, sulfamyl, cyano or nitro,

a) X denotes oxygen,
Y denotes a single bond and
Z denotes a straight-chain or branched alkylene chain having 2 to 5 carbon atoms, which is optionally substituted with a hydroxy group, or

b) X, Y and Z denote a single bond, or

c) X denotes the —NR— group, in which R denotes hydrogen or $C_1$-$C_4$-alkyl,
Y denotes a single bond and
Z denotes a straight-chain or branched alkylene chain having 2 to 5 carbon atoms, or

d) X denotes the —NR— group, in which R denotes hydrogen or $C_1$-$C_4$-alkyl,
Y denotes the carbonyl, thiocarbonyl or iminocarbonyl group and
Z denotes a single bond, or

e) X denotes the —NR— group, in which R denotes hydrogen or $C_1$-$C_4$-alkyl,
Y denotes the carboxamido, thiocarboxamido or carboxamidino group and
Z denotes a straight-chain or branched alkylene chain having 2 to 5 carbon atoms, or

f) X denotes the —NR— group, in which R denotes hydrogen or $C_1$-$C_4$-alkyl,
Y denotes the carbonyl group and
Z denotes a straight-chain or branched alkylene chain having 1 to 3 carbon atoms, or

g) X denotes the —NR— group, in which R denotes hydrogen or $C_1$-$C_4$-alkyl,
Y denotes the carboxyl group and

Z denotes a straight-chain or branched alkylene chain having 2 to 5 carbon atoms, and

$R^4$ and $R^5$ are identical or different and denote hydrogen or straight-chain or branched $C_1$-$C_4$-alkyl, whereby $R^4$ and

$R^5$ are not hydrogen at the same time, or $R^4$ and $R^5$ together are an alkylene chain having 4 to 6 carbon atoms which form, with the nitrogen atom, a 5- to 7-membered heterocyclic ring, in which it is possible for one $—CH_2—$ group to be replaced by another heteroatom from the group comprising nitrogen, oxygen or sulfur, and it being possible for the heterocylic ring in turn to be substituted with an alkyl group having one to three carbon atoms which is optionally substituted with a hydroxyl group, or with phenyl-$C_1$-$C_3$-alkyl or naphthyl-$C_1$-$C_3$-alkyl or with phenyl or naphthyl, which is optionally substituted with methyl, methoxy, chlorine or trifluoromethyl, and

$R^6$ denotes hydrogen or the hydroxyl group, and their physiologically tolerated acid addition and ammonium salts, characterized in reacting 3-aryl-7-chloro-3,4-dihydroacridine-1,9-(2H, 10H)-dione of the formula II

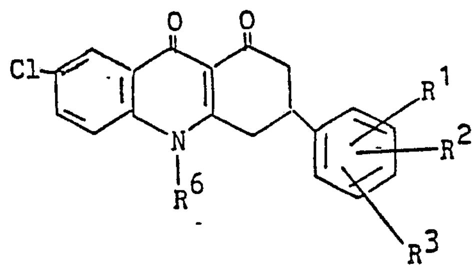

(II)

in which $R^1$, $R^2$, $R^3$ and $R^6$ have the meaning indicated for formula I, with a compound of the formula III

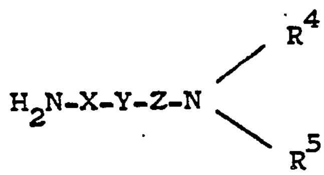

(III)

in which X, Y, Z and $R^4$ and $R^5$ have the meaning indicated for formula I, or with their salts, and converting the reaction product, where appropriate, with a physiologically tolerated acid into an addition salt or with an alkylating agent into an ammonium salt.

2. A process as claimed in claim 1, characterized in reacting such compounds of formula III, in which $R^4$ and $R^5$ are different from hydrogen.

3. A process as claimed in claim 2, characterized in reacting such compounds of formula II, in which $R^1$ and $R^2$ are hydrogen and $R^3$ is chlorine or trifluoromethyl, with such compounds of formula III, in which X, Y and Z denote a single bond and $R^4$ and $R^5$ denote, together with the nitrogen atom, a piperazine ring, which is optionally substituted on the nitrogen.

4. A process as claimed in claim 2, characterized in reacting the 3-(4-trifluoromethylphenyl)-7-chloro-3,4-dihydroacridine-1,9-(2H, 10H)-dione with N-methyl-N'-aminopiperazin or N-2-hydroxyethyl-N'-aminopiperazin, respectively.

5. A process as claimed in claim 2, characterized in reacting 3-(4-chlorophenyl)-7-chloro-3,4-dihydroacridine-1,9-(2H, 10H)-dione with N-methyl-N'-aminopiperazin or N-2-hydroxyethyl-N'-aminopiperazin respectively.

**Revendications** (pour les Etats contractants : BE, CH, DE, FR, GB, IT, LI, LU, NL, SE)

1. Des 3-aryl-7-chloro-3,4-dihydro-acridine-1,9-(2H, 10H)-dione-1-oximes et 1-hydrazones de formule I

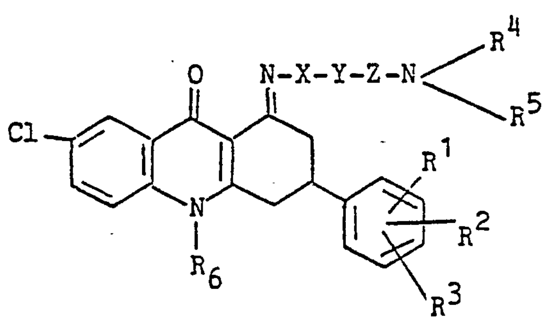

(I)

dans laquelle

$R^1$, $R^2$ et $R^3$ sont identiques ou différents et représentent l'hydrogène, un halogène, un groupe alcoyle en $C_1$-$C_4$, trifluorométhyle, alcoxy en $C_1$-$C_4$, trifluorométhoxy, difluorochlorométhoxy, 1,1,2,2-tétrafluoroéthoxy, phénoxy, halogénophénoxy, un groupe alcoyl-($C_1$-$C_4$)-, phényl- et naphtyl-thio, un groupe alcoyl-($C_1$-$C_4$)-, phényl- et napthtyl -sulfinyle, un groupe alcoyl-($C_1$-$C_4$)-, phényl- et naphtyl-sulfonyle, un groupe trifluorométhylthio, carbamyle, sulfamyle, cyano ou nitro,

a) X représente l'oxygène,

Y représente une simple liaison,

Z représente une chaîne alcoylène droite ou ramifiée, ayant de 2 à 5 atomes de carbone, éventuellement substituée par un groupe hydroxy, ou

b) X, Y et Z représentent une simple liaison, ou

c) X représente le groupe —NR— dans lequel R est l'hydrogène ou un groupe alcoyle en $C_1$-$C_4$,

Y représente une simple liaison,

Z représente une chaîne alcoylène droite ou ramifiée, ayant de 2 à 5 atomes de carbone, ou

d) X représente le groupe —NR— dans lequel R est l'hydrogène ou un groupe alcoyle en $C_1$-$C_4$,

Y représente un groupe carbonyle, thiocarbonyle ou iminocarbonyle,

Z représente une simple liaison, ou

e) X représente un groupe —NR— dans lequel R est l'hydrogène ou un groupe alcoyle en $C_1$-$C_4$,

Y représente un groupe carbonamide, thiocarbonamide ou carboamidine,

Z représente une chaîne alcoylène droite ou ramifiée, ayant de 2 à 5 atomes de carbone, ou

f) X représente le groupe —NR— dans lequel R est l'hydrogène ou un groupe alcoyle en $C_1$-$C_4$,

Y représente un groupe carbonyle,

Z représente une chaîne alcoylène droite ou ramifiée ayant de 1 à 3 atomes de carbone, ou

g) X représente le groupe —NR— dans lequel R est l'hydrogène ou un groupe alcoyle en $C_1$-$C_4$,

Y représente un groupe carboxy,

Z représente une chaîne alcoylène droite ou ramifiée ayant de 2 à 5 atomes de carbone, et

$R^4$ et $R^5$ sont identiques ou différents et représentent l'hydrogène ou un groupe alcoyle à chaîne droite ou ramifiée en $C_1$-$C_4$, $R^4$ et $R^5$ n'étant pas simultanément l'hydrogène, ou $R^4$ et $R^5$ forment ensemble une chaîne alcoylène ayant de 4 à 6 atomes de carbone qui forme avec l'atome d'azote un noyau hétérocyclique de 5 à 7 chaînons, dans lequel un groupe —$CH_2$— peut être remplacé par un autre hétéroatome choisi parmi l'azote, l'oxygène ou le soufre, et où le noyau hétérocyclique peut de son côté être substitué par un groupe alcoyle ayant de 1 à 3 atomes de carbone, éventuellement substitué avec un groupe hydroxy, ou par un groupe phényl- ou naphtyl-, alcoyle en $C_1$-$C_3$ ou phényle ou naphtyle éventuellement substitué par un groupe méthyle, méthoxy, le chlore ou un groupe trifluorométhyle, et $R_6$ représente l'hydrogène ou le groupe hydroxy, ainsi que leurs sels d'addition acides et leurs sels d'ammonium physiologiquement tolérés.

2. Des composés tels que définis sous 1, caractérisés en ce que $R^4$ et $R^5$ sont différents de l'hydrogène.

3. Des composés tels que définis sous 2, caractérisés en ce que $R^1$ et $R^2$ représentent l'hydrogène, $R^3$ représente le chlore ou un groupe trifluorométhyle, X, Y et Z représentent une simple liaison et $R^4$ et $R^5$ représentent ensemble avec l'atome d'azote un noyau pipérazine qui est éventuellement substitué sur l'azote.

4. Un composé tel que défini sous 2, caractérisé en ce qu'il s'agit de la 3-(4-trifluorométhylphényl)-7-chloro-3,4-dihydroacridine-1,9-(2H, 10H)-dione-1-(N-méthyl-N'-pipérazine)-imine ou de la 3-(4-trifluorométhylphényl)-7-chloro-3,4-dihydroacridine-1,9-(2H, 10H)-dione-1-N-2-hydroxyéthyl-N'-pipérazinimine, respectivement.

5. Un composé tel que défini sous 2, caractérisé en ce qu'il s'agit de la 3-(4-chlorophényl)-7-chloro-3,4-dihydro-acridine-1,9-(2H, 10H)-dione-1-N-méthyl-N'-pipérazinimine ou de la 3-(4-chlorophényl)-7-chloro-3,4-dihydro-acridine-1,9-(2H, 10H)-dione-1-N-2-hydroxyéthyl-N'-pipérazinimine, respectivement.

6. Un procédé pour la préparation de 3-aryl-7-chloro-3,4-dihydro-acridine-1,9-(2H, 10H)-dione-1-oximes et 1-hydrazones telles que définies sous 1, caractérisé en ce qu'on fait réagir une 3-aryl-7-chloro-3,4-dihydro-acridine-1,9-(2H, 10H)-dione de formule II

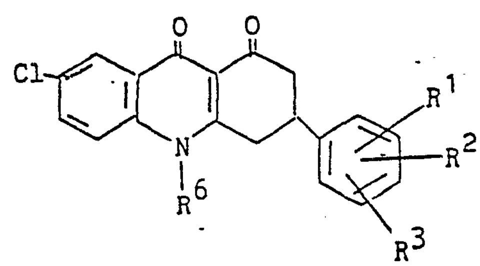

(II)

dans laquelle $R^1$, $R^2$, $R^3$ et $R^6$ ont les significations indiquées pour la formule I, avec un composé de formule III

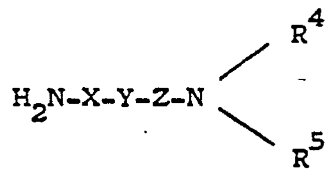

(III)

dans laquelle X, Y, Z ainsi que $R^4$ et $R^5$ ont les significations indiquées pour la formule I, ou avec ses sels, et on convertit éventuellement le produit de réaction en un sel d'addition avec un acide physiologiquement acceptable ou en un sel d'ammonium avec un agent d'alcoylation.

7. Des compositions pharmaceutiques contenant un composé tel que défini dans une ou plusieurs des revendications 1 à 5.

8. Un procédé pour la préparation d'une composition pharmaceutique telle que définie sous 7, caractérisé en ce qu'on met un composé tel que défini dans une ou plusieurs des revendications 1 à 5 sous une forme d'administration appropriée.

9. Des composés tels que définis dans une ou plusieurs des revendications 1 à 5 ou une composition pharmaceutique telle que définie sous 7 pour lutter contre des infections dues aux protozoaires chez l'homme et l'animal.

**Revendications** (pour l'Etat contractant AT)

1. Procédé de préparation de 3-aryl-7-chloro-3,4-dihydro-acridine-1,9-(2H, 10H)-dione-1-oximes et 1-hydrazones de formule I

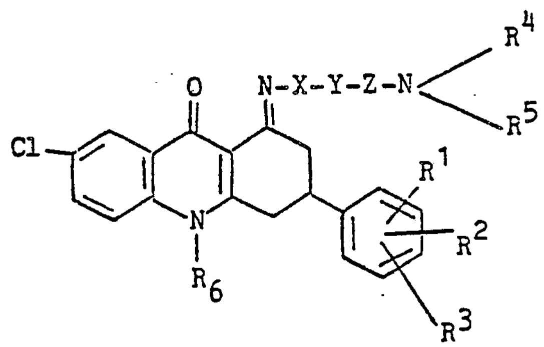

(I)

dans laquelle

$R^1$, $R^2$ et $R^3$ sont identiques ou différents et représentent l'hydrogène, un halogène, un groupe alcoyle en $C_1$-$C_4$, trifluorométhyle, alcoxy en $C_1$-$C_4$, trifluorométhoxy, difluorochlorométhoxy, 1,1,2,2-tétrafluoroéthoxy, phénoxy, halogénophénoxy, un groupe alcoyl-($C_1$-$C_4$)-, phényl- et naphtyl-thio, un groupe alcoyl-($C_1$-$C_4$)-, phényl- et naphtyl -sulfinyle, un groupe alcoyl-($C_1$-$C_4$)-, phényl- et naphtyl-sulfonyle, un groupe trifluorométhylthio, carbamyle, sulfamyle, cyano ou nitro,

a) X représente l'oxygène,
Y représente une simple liaison,
Z représente une chaîne alcoylène droite ou ramifiée, ayant de 2 à 5 atomes de carbone, éventuellement substituée par un groupe hydroxy, ou

b) X, Y et Z représentent une simple liaison, ou

c) X représente le groupe —NR— dans lequel R est l'hydrogène ou un groupe alcoyle en $C_1$-$C_4$,
Y représente une simple liaison,
Z représente une chaîne alcoylène droite ou ramifiée, ayant de 2 à 5 atomes de carbone, ou

d) X représente le groupe —NR— dans lequel R est l'hydrogène ou un groupe alcoyle en $C_1$-$C_4$,
Y représente un groupe carbonyle, thiocarbonyle ou iminocarbonyle,
Z représente une simple liaison, ou

e) X représente un groupe —NR— dans lequel R est l'hydrogène ou un groupe alcoyle en $C_1$-$C_4$,
Y représente un groupe carbonamide, thiocarbonamide ou carboamidine,
Z représente une chaîne alcoylène droite ou ramifiée, ayant de 2 à 5 atomes de carbone, ou

f) X représente le groupe —NR— dans lequel R est l'hydrogène ou un groupe alcoyle en $C_1$-$C_4$,

Y représente un groupe carbonyle,

Z représente une chaîne alcoylène droite ou ramifiée ayant de 1 à 3 atomes de carbone, ou

g) X représente le groupe —NR— dans lequel R est l'hydrogène ou un groupe alcoyle en $C_1$-$C_4$,

Y représente un groupe carboxy,

Z représente une chaîne alcoylène droite ou ramifiée ayant de 2 à 5 atomes de carbone et

$R^4$ et $R^5$ sont identiques ou différents et représentent l'hydrogène ou un groupe alcoyle à chaîne droite ou ramifiée en $C_1$-$C_4$, $R^4$ et $R^5$ n'étant pas simultanément l'hydrogène, ou $R^4$ et $R^5$ forment ensemble une chaîne alcoylène ayant de 4 à 6 atomes de carbone qui forme avec l'atome d'azote un noyau hétérocyclique de 5 à 7 chaînons, dans lequel un groupe —$CH_2$— peut être remplacé par un autre hétéroatome choisi parmi l'azote, l'oxygène ou le soufre, et où le noyau hétérocyclique peut de son côté être substitué par un groupe alcoyle ayant de 1 à 3 atomes de carbone, éventuellement substitué avec un groupe hydroxy, ou par un groupe phényl- ou naphtyl-, alcoyle en $C_1$-$C_3$ ou phényle ou naphtyle éventuellement substitué par un groupe méthyle, méthoxy, le chlore ou un groupe trifluorométhyle, et

$R^6$ représente l'hydrogène ou un groupe hydroxy, ainsi que de leurs sels d'addition avec des acides physiologiquement acceptables ou leurs sels d'ammonium, caractérisé en ce qu'on fait réagir une 3-aryl-7-chloro-3,4-dihydro-acridine-1,9-(2H, 10H)-dione de formule II

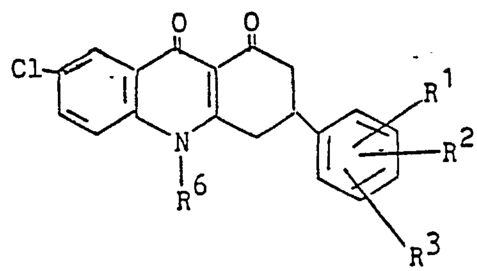

(II)

dans laquelle $R^1$, $R^2$, $R^3$ et $R^6$ ont les significations indiquées pour la formule I, avec un composé de formule III

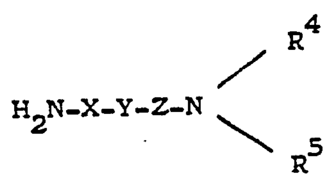

dans laquelle X, Y, Z ainsi que $R^4$ et $R^5$ ont les significations indiquées pour la formule I, ou avec ses sels, et on convertit éventuellement le produit de réaction en un sel d'addition avec un acide physiologiquement acceptable ou en un sel d'ammonium avec un agent d'alcoylation.

2. Procédé selon la revendication 1, caractérisé en ce que l'on fait réagir des composés répondant à la formule III, dans lesquels R4 et $R^5$ sont différents de l'hydrogène.

3. Procédé selon la revendication 2, caractérisé en ce que $R^1$ et $R^2$ représentent l'hydrogène, $R^3$ représente le chlore ou un groupe trifluorométhyle, X, Y et Z représentent une simple liaison et $R^4$ et $R^5$ représentent ensemble avec l'atome d'azote un noyau pipérazine qui est éventuellement substitué sur l'azote.

4. Procédé selon la revendication 2, caractérisé en ce qu'on fait réagir la 3-(4-trifluorométhylphényl)-7-chloro-3,4-dihydro-acridine-1,9-(2H, 10H)-dione-1-(N-méthyl-N'-pipérazine)-imine ou la 3-(4-trifluorométhylphényl)-7-chloro-3,4-dihydro-acridine-1,9-(2H, 10H)-dione-1-N-2-hydroxyéthyl-N'-pipérazinimine, respectivement.

5. Procédé selon la revendication 2, caractérisé en ce qu'on fait réagir la 3-(4-chlorophényl)-7-chloro-3,4-dihydroacridine-1,9-(2H, 10H)-dione-1-N-méthyl-N'-pipérazinimine ou la 3-(4-chlorophényl)-7-chloro-3,4-dihydro-acridine-1,9-(2H, 10H)-dione-1-N-2-hydroxyéthyl-N'-pipérazinimine, respectivement.